# EUROPEAN PATENT APPLICATION

(11) **EP 4 768 034 A2**
(43) Date of publication of application: **01.07.2026**
(21) Application number: 25227226.5
(22) Date of filing: 24.12.2025
(51) Int. Cl.: A61K 35/74, A61K 35/741, A61K 35/747, A61K 39/395, C07K 16/30, A61K 31/195, A61K 31/404, A61K 31/66, A61K 31/685, A61K 45/06, A61P 35/00, A61P 1/00

(54) **USE OF GUT MICROBIOTA OR METABOLITE THEREOF AND ANTIBODY OR DERIVATIVE THEREOF IN PREPARATION OF DRUG FOR IMMUNOTHERAPY OF COLORECTAL CANCER (CRC)**

(30) Priority: 04.03.2025 CN 202510252535; 25.12.2024 CN 202411935089
(71) Applicant: Guangzhou Juncai Jifang Biotechnology Co., Ltd., Guangzhou, Guangdong 510000 (CN)
(72) Inventor: HUANG, Jun, Guangzhou, 510655 (CN); YAO, Qijun, Guangzhou, 510655 (CN); PEI, Fengyun, Guangzhou (CN)
(74) Representative: Metida

(57) **Abstract**

Provided is at least one agent selected from the group consisting of (a) a gut microbiota or a metabolite thereof and (b) an antibody or a derivative thereof for use in immunotherapy of colorectal cancer. The gut microbiota includes at least one selected from the group consisting of Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium, and Alistipes onderdonkii. The gut microbiota or the metabolite thereof and/or the antibody or the derivative thereof in this application can enhance the anti-tumor effect of immunotherapy, and demonstrate the functions of regulating the immune microenvironment and improving sensitivity to tumor immunotherapy.

## Description

### TECHNICAL FIELD

The present application relates to the field of biotechnologies, and in particular, relates to a use of a gut microbiota or a metabolite thereof and/or an antibody or a derivative thereof in preparation of a drug for immunotherapy of colorectal cancer (CRC).

### BACKGROUND

Colorectal cancer (CRC) is one of the three most common malignant tumors worldwide and ranks fourth in terms of mortality among malignant tumors. Microsatellites (MSs) are short tandem DNA repeats each composed of 1 to 6 nucleotides in the genome. MSs are widely distributed in both coding and noncoding regions of the human genome. MSs are extremely sensitive to DNA mismatches due to their high repeatability, and can reflect tumor-associated genomic instability. The phenomenon where a new MS allele appears in a tumor due to the insertion or deletion of a repeat unit in MS is referred to as microsatellite instability (MSI). Conversely, the absence of such alterations is referred to as microsatellite stability (MSS).

Immunotherapy, as a novel treatment approach for malignant tumors, represents a milestone innovation in the history of malignant tumor therapy. T cells are the core executors of anti-tumor immunity in the body. The activation of T cells requires the following two key signals: a first signal resulting from the binding of the major histocompatibility complex-antigen peptide on the surface of antigen-presenting cells to the T cell receptor; and a second signal resulting from the interaction between costimulatory molecules on the surface of antigen-presenting cells and costimulatory molecules on the surface of T cells. The costimulatory molecules include positive and negative molecules. The positive molecules promote the proliferation and differentiation of lymphocytes and the secretion of cytokines. The negative molecules weaken, restrict, and terminate the immune response of lymphocytes. Immune checkpoints refer to key factors expressed on immune cells, which can regulate the degree of immune activation. Immune checkpoint pathways are regulated through ligand-receptor interactions. Under physiological conditions, immune checkpoints play a crucial role in maintaining the self-tolerance, regulating the duration of a physiological immune response, etc. Based on this characteristic of the immune system, tumor cells can overexpress ligands for immune checkpoint pathways to inhibit the activity of T cells, and thus evade immune surveillance and cytotoxic effects, which promotes the growth of tumor cells. Currently identified inhibitory receptors include cytotoxic T lymphocyte antigen-4 (CTLA-4), programmed cell death protein 1 (PD-1) and programmed cell death-ligand 1 (PD-L1), lymphocyte activation gene-3 (LAG-3), and T-cell immunoglobulin and mucin-domain-containing molecule-3 (TIM-3), etc.

Immunotherapy, represented by immune checkpoint inhibitors (ICIs), has been proved by an increasing number of clinical studies to demonstrate excellent efficacy in patients with microsatellite instability-high (MSI-H) CRC. However, immunotherapy remains largely ineffective in MSS CRC patients, who account for up to 90% of CRC patients. MSS CRC patients acquire almost no benefit from ICI monotherapy. Currently, the clinical exploration for MSS/mismatch repair-proficient (pMMR) non-metastatic CRC primarily focuses on the combination of immunotherapy with chemotherapy, the combination of immunotherapy with radiotherapy, the combination of immunotherapy with targeted therapy, and dual immunotherapy, but successful cases remain relatively limited. The combination, administration sequence, and doses of drugs for combination therapy regimens still warrant further exploration. In view of the aforementioned clinical treatment bottleneck, the applicant has pioneered a neoadjuvant therapy regimen combining mFOLFOX6, bevacizumab, and an anti-PD-1 monoclonal antibody for MSS CRC patients. This neoadjuvant therapy regimen has also been registered as a prospective clinical trial (NCT04895137) and has been granted a Chinese invention patent (CN202210083399.9, granted as CN114522227B) and a U.S. invention patent (US18/099291, granted as US12053472B2). The applicant intends to enhance the sensitivity of MSS CRC patients to immunotherapy through this novel treatment regimen, and to avoid the toxic and side effects associated with triplet chemotherapy and concurrent chemoradiotherapy while striving to maximize the R0 resection rate and pathological complete response (pCR). Compared to the FOXTROT and OPTICAL clinical studies, the applicant has increased the pCR of neoadjuvant chemotherapy for colon cancer from 4% to 20.5%. Further, up to 64.1% of MSS CRC patients are sensitive to this neoadjuvant therapy regimen, achieving major pathological response (MPR). Nevertheless, it still fails to achieve MPR in a small proportion of MSS CRC patients who are insensitive to the treatment. Therefore, immunotherapy for MSS/pMMR CRC may require the selection of potential benefiting populations, and accordingly, targeted combination therapy should be implemented based on immunophenotyping and relevant molecular subtyping.

Current strategies for improving the sensitivity to immunotherapy may involve complex gene editing, targeted drugs, or chemotherapy, which are invasive and accompanied by significant side effects. Some of the existing immune enhancement therapies, such as cell therapy and gene therapy, require complex production processes and high costs, which limits the widespread application. Due to the unique anatomical and pathophysiological characteristics of CRC, the role of gut microbiota and metabolites thereof in the development and progression of CRC has garnered extensive attention over the past decade. As the largest immune organ in the body, the gastrointestinal tract harbors 60% to 80% of immune cells in the body and includes immune-related structures maintaining immune homeostasis. The gut microbiota and metabolites thereof can influence both adaptive immunity and innate immunity through multiple pathways, and exert intricate immunomodulatory effects on systemic tumor immunity. However, there have been no studies focusing on the enhancement of immunotherapy sensitivity in CRC based on gut microbiota.

### SUMMARY

The present application discloses at least one agent selected from the group consisting of: (a) a gut microbiota or a metabolite thereof, and (b) an antibody or a derivative thereof, for use in immunotherapy of colorectal cancer (CRC), where the gut microbiota includes at least one selected from the group consisting of *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii;* and the antibody includes a monoclonal antibody targeting an interleukin-17A (IL-17A) signaling pathway, a monoclonal antibody targeting CRC, or a combination thereof.

The present application discloses a composition for use in immunotherapy of CRC, where the composition includes: (1) the at least one agent selected from the group consisting of (a) a gut microbiota or a metabolite thereof and (b) an antibody or a derivative thereof (abbreviated as "the at least one agent"); and (2) a product for treating CRC.

The present application discloses the at least one agent for use in improving or predicting sensitivity to an immune checkpoint inhibitor.

The present application discloses the at least one agent for use in inhibiting an IL-17A signaling pathway.

In some embodiments of the composition for use, the at least one agent includes the monoclonal antibody targeting the IL-17A signaling pathway, and the product for treating CRC includes an immune checkpoint inhibitor.

The present application discloses a pharmaceutical composition, including: (1) at least one agent ; and (2) a product for treating CRC.

The present application discloses the composition for use or the pharmaceutical composition for use, where the product for treating CRC includes an immune checkpoint inhibitor. In some embodiments, the increasing sensitivity of CRC to an immunotherapy drug includes increasing sensitivity to the immune checkpoint inhibitor. In some embodiments, the increasing sensitivity of CRC to an immunotherapy drug further includes increasing sensitivity to an immune cell therapy drug and/or a cytokine therapy drug.

In some embodiments, the product for treating CRC includes at least one selected from the group consisting of a microbial strain, an immune checkpoint inhibitor, an immune activator, a cancer vaccine, an immune cell therapy drug, and a cytokine therapy drug.

In some embodiments, the gut microbiota includes at least two selected from the group consisting of *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii.*

In some embodiments, the metabolite of the gut microbiota includes at least one selected from the group consisting of O-phosphorylethanolamine, 5,6-dihydroxyindole-2-carboxylic acid, phosphatidylcholine (O-16:0_20:4), and 4-acetamidobutyric acid.

In some embodiments, a metabolite of *Alistipes shahii* includes at least one selected from the group consisting of O-phosphorylethanolamine, 5,6-dihydroxyindole-2-carboxylic acid, and phosphatidylcholine (O-16:0_20:4). In some embodiments, a metabolite of *Butyricimonas virosa* includes 4-acetamidobutyric acid.

In some embodiments, the product for treating CRC includes an immune checkpoint inhibitor.

In some embodiments, the immune checkpoint inhibitor includes an anti-PD-1 antibody, an anti-PD-L1 antibody, or a combination thereof.

In some embodiments, the product for treating CRC includes a microbial strain.

In some embodiments, the microbial strain includes at least one selected from the group consisting of *Lactobacillus rhamnosus, Lactobacillus reuteri, Lactobacillus johnsonii,* and *Bifidobacterium.*

In some embodiments, the CRC includes MSS CRC and MSI CRC.

In some embodiments, dosage forms of (1) the at least one agent and optionally (2) the product for treating CRC are each independently selected from the group consisting of liquid, gas, solid, and semi-solid dosage forms.

In some embodiments, the dosage form further includes a pharmaceutically acceptable excipient.

### BRIEF DESCRIPTION OF THE DRAWINGS

FIG. 1, together with FIG. 1 (continuous 1), FIG. 1 (continuous 2), FIG. 1 (continuous 3), and FIG. 1 (continuous 4), form a complete figure, and a combination of these sub-figures shows linear discriminant analysis effect size (LEfSe) analysis results of species from fecal metagenomic sequencing in MSS CRC patients before treatment, stratified by their sensitivity to neoadjuvant immunotherapy;
FIG. 2A, FIG. 2B, FIG. 2C, and FIG. 2D respectively shows analysis results of a tumor size, a tumor volume, a body weight, and a tumor weight of subcutaneous MSI-H CRC mouse models from different treatment groups (including *Alistipes shahii)* in Example 2;
FIG. 3A, FIG. 3B, FIG. 3C, and FIG. 3D respectively shows analysis results of a tumor size, a tumor volume, a body weight, and a tumor weight of subcutaneous MSS CRC mouse models from different treatment groups (including *Alistipes shahii)* in Example 2;
FIG. 4 shows analysis results of the infiltration of Th17 cells and effector CD8⁺ T cells (Granzyme B (GZMB⁺) CD8⁺ T cells) in tumors of orthotopic MSS CRC mouse models from different treatment groups (including *Alistipes shahii)* in Example 2;
FIG. 5A and FIG. 5B respectively shows a bar chart of the infiltration of Th17 cells and effector CD8⁺ T cells (GAMB⁺ CD8⁺ T cells) in tumors of orthotopic MSS CRC mouse models from different treatment groups (including *Alistipes shahii)* in Example 2;
FIG. 6 shows analysis results of the infiltration of CD8⁺ T cells in tumors of orthotopic MSS CRC mouse models from different treatment groups (including *Alistipes shahii)* in Example 2;
FIG. 7A shows the infiltration of exhausted immune cells (PD-1⁺ cells) in tumors of orthotopic MSS CRC mouse models from different treatment groups (including *Alistipes shahii)* in Example 2, and FIG. 7B shows analysis results thereof;
FIG. 8A and FIG. 8B respectively shows *in vivo* imaging results and tumor fluorescence intensity analysis results of orthotopic MSS CRC mouse models from different treatment groups (including *Alistipes shahii)* in Example 2 (a fluorescence intensity is proportional to a number and activity of tumor cells), where days are recorded as follows: the first day after tumor inoculation is recorded as Day 1, and subsequent days are recorded accordingly;
FIG. 9A, FIG. 9B, and FIG. 9C respectively shows analysis results of a tumor size, a tumor volume, and a tumor weight of orthotopic MSS CRC mouse models from different treatment groups (including *Alistipes shahii)* in Example 2;
FIG. 10A, FIG. 10B, and FIG. 10C respectively shows analysis results of a tumor size, a tumor volume, and a tumor weight of subcutaneous MSS CRC mouse models from different treatment groups (including *Butyricimonas virosa)* in Example 2;
FIG. 11A, FIG. 11B, FIG. 11C, FIG. 11D, and FIG. 11E respectively shows analysis results of a tumor fluorescence intensity, total flux, a tumor size, a tumor volume, and a tumor weight of orthotopic MSS CRC mouse models from different treatment groups (including *Butyricimonas virosa)* in Example 2;
FIG. 12A shows significant enrichment results of phosphatidylcholine (O-16:0_20:4) in intestinal contents of mice from a phosphate buffered saline (PBS) group and an *Alistipes shahii* group; FIG. 12B shows significant enrichment results of phosphatidylcholine (0-16:0_20:4) in intestinal contents of mice from an anti-PD-1 monoclonal antibody group, and anti-PD-1 monoclonal *antibody*/*Alistipes shahii* combined administration group in Example 3;
FIG. 13A and FIG. 13C respectively shows significant enrichment results of O-phosphorylethanolamine in culture supernatants from the *Alistipes shahii* group and the *Alistipes* shahii/anti-PD-1 monoclonal antibody combined administration group in Example 3; FIG. 13B and FIG. 13D respectively shows secondary mass spectra of O-phosphorylethanolamine obtained from full-spectrum metabolomics PLUS analysis of these groups;
FIG. 14 shows significant enrichment results of 5,6-dihydroxyindole-2-carboxylic acid in an intestinal content of the *Alistipes shahii*/*anti-PD-1* monoclonal antibody combined administration group in Example 3;
FIG. 15 shows significant enrichment results of 4-acetamidobutyric acid in an intestinal content of mice from a *Butyricimonas* virosa/anti-PD-1 monoclonal antibody combined administration group in Example 3;
FIG. 16A and FIG. 16B both shows KEGG enrichment analysis results of bulk transcriptomes from tumor tissues in Example 4; (FIG. 16A shows the significant down-regulation of IL-17 signaling pathway in pre-treatment tumor tissues of MPR patients compared to non-MPR patients; and FIG. 16B shows the significant down-regulation of both IL-17 signaling pathway and Th17 cell differentiation pathway in post-treatment tumor tissues of MPR patients compared to non-MPR patients);
FIG. 17A and FIG. 17B both shows gene set enrichment analysis (GSEA) results of bulk transcriptomes from pre-treatment tumor tissues in Example 4 (FIG. 17A shows results of IL-17 signaling pathway; and FIG. 17B shows results of Th17 cell differentiation pathway);
FIG. 18A and FIG. 18B both shows GSEA results of bulk transcriptomes from post-treatment tumor tissues in Example 4 (FIG. 18A shows results of IL-17 signaling pathway; and FIG. 18B shows results of Th17 cell differentiation pathway);
FIG. 19 shows flow cytometry results of Th17 cell differentiation under different treatment conditions (part I);
FIG. 20 shows flow cytometry results of Th17 cell differentiation under different treatment conditions (part II);
FIG. 21A shows a bar chart of Th17 cell differentiation before and after inducing differentiation; FIG. 21B shows a bar chart of Th17 cell differentiation under different treatment conditions;
FIG. 22 shows tumors of subcutaneous MSS CRC mouse models from different treatment groups (including O-phosphorylethanolamine and 5,6-dihydroxyindole-2-carboxylic acid) in Example 6;
FIG. 23A and FIG. 23B respectively shows analysis results of a tumor volume and a tumor weight of subcutaneous MSS CRC mouse models from different treatment groups (including O-phosphorylethanolamine and 5,6-dihydroxyindole-2-carboxylic acid) in Example 6;
FIG. 24A, FIG. 24B, and FIG. 24C respectively shows analysis results of the infiltration of Th17 cells, CD8⁺ T cells, and effector CD8⁺ T cells (GZMB⁺ CD8⁺ T cells) in tumors of subcutaneous MSS CRC mouse models from different treatment groups (including O-phosphorylethanolamine and 5,6-dihydroxyindole-2-carboxylic acid) in Example 6;
FIG. 25A, FIG. 25B, and FIG. 25C respectively shows a bar chart of the infiltration of Th17 cells, CD8⁺ T cells, and effector CD8⁺ T cells (GZMB⁺ CD8⁺ T cells) in tumors of subcutaneous MSS CRC mouse models from different treatment groups (including O-phosphorylethanolamine and 5,6-dihydroxyindole-2-carboxylic acid) in Example 6; and
FIG. 26A shows the infiltration of exhausted immune cells (PD-1⁺ cells) in tumors of subcutaneous MSS CRC mouse models from different treatment groups (including O-phosphorylethanolamine and 5,6-dihydroxyindole-2-carboxylic acid) in Example 6; FIG. 26B shows analysis results thereof.

### DETAILED DESCRIPTION

The present application provides a use of a gut microbiota or a metabolite thereof and/or an antibody or a derivative thereof in preparation of a drug for immunotherapy of CRC. The gut microbiota or the metabolite thereof and/or the antibody or the derivative thereof in the present application can enhance the sensitivity of CRC patients to immunotherapy. The gut microbiota or the metabolite thereof and/or the antibody or the derivative thereof can also predict the survival, prognosis, and sensitivity to immunotherapy for MSS CRC patients.

The present application adopts the following technical solutions:

The present application provides a use of at least one selected from the group consisting of (a) a gut microbiota or a metabolite thereof and (b) an antibody or a derivative thereof in preparation of a drug for immunotherapy of CRC; the gut microbiota includes at least one selected from the group consisting of *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii;*
the antibody includes a monoclonal antibody targeting an IL-17A signaling pathway, a monoclonal antibody targeting CRC, or a combination thereof.

In the present application, based on the prospective clinical trial of the applicant, gut microbiota in fecal samples from MSS CRC patients sensitive to immunotherapy (MPR) and those insensitive to immunotherapy (non-MPR) are compared, and 10 bacterial species enriched in fecal samples of MPR patients are discovered and identified. The pathogenicity of these 10 bacterial species *(Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii)* has not been reported.

Data of the prospective clinical trial has proved that the novel drug combination (mFOLFOX6 + bevacizumab + anti-PD-1 monoclonal antibody) of the applicant can significantly enhance the sensitivity of MSS CRC to immunotherapy, maximize the R0 resection rate and pCR, and avoid the toxic and side effects associated with triplet chemotherapy and concurrent chemoradiotherapy. Although this regimen can remarkably enhance the immunotherapy sensitivity, pCR, and MPR in MSS CRC patients, some patients remain insensitive to the treatment and fails to achieve MPR.

The discovery of the aforementioned bacterial species is expected to further enhance the therapeutic efficacy and prognosis for insensitive patients, thereby improving the overall survival of CRC patients (particularly MSS CRC patients). These species may serve as targets for clinically predicting and improving the sensitivity of CRC to immunotherapy, such that patients can receive accurate neoadjuvant therapy.

Moreover, the present application has discovered that the metabolite of the gut microbiota can also enhance the sensitivity of CRC patients to immunotherapy and predict the survival, prognosis, and immunotherapy sensitivity in CRC patients (particularly MSS CRC patients).

The present application has also discovered that *Alistipes shahii* can secrete related metabolites to inhibit the differentiation of naive CD4⁺ T cells into Th17 cells and reduce the infiltration and functionality of Th17 cells in CRC, thereby enhancing the therapeutic efficacy of the anti-PD-1 monoclonal antibody for CRC. IL-17A is primarily secreted by Th17 cells. A monoclonal antibody targeting an IL-17A signaling pathway can effectively diminish Th17 cells and IL-17A secreted thereby, consequently achieving the therapeutic efficacy against CRC.

The gut microbiota (such as *Alistipes shahii)* can also significantly reduce the infiltration of Th17 cells and exhausted immune cells (PD-1⁺ cells) in the tumor and increase the CD8⁺ T cells and effector CD8⁺ T cells (GZMB⁺ CD8⁺ T cells) infiltrating the tumor. The combined use of the gut microbiota *(Alistipes shahii)* with the anti-PD-1 monoclonal antibody can further enhance this effect.

The combined use of the anti-IL-17A monoclonal antibody with the anti-PD-1 monoclonal antibody can also significantly reduce the infiltration of Th17 cells and exhausted immune cells (PD-1⁺ cells) in the tumor and increase the CD8⁺ T cells and effector CD8⁺ T cells (GZMB⁺ CD8⁺ T cells) infiltrating the tumor. Therefore, the gut microbiota (such as *Alistipes shahii)* may suppress the infiltration of Th17 cells in the tumor to promote the infiltration and functionality of CD8⁺ T cells in the tumor, thereby facilitating an anti-tumor immune response.

In some embodiments, the gut microbiota includes at least two selected from the group consisting of *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii.*

The combined use of any of the aforementioned gut microbiota species in the present application can also enhance the sensitivity of mice to CRC immunotherapy drugs, and can also achieves significant anti-tumor effects (applicable for CRC therapy).

In some specific embodiments, combinations of gut microbiota species (pairwise combinations) may include the following: 1) *Alistipes shahii* and *Butyricimonas virosa;* 2) *Alistipes shahii* and *Desulfovibrio piger;* 3) *Alistipes shahii* and *Alistipes putredinis;* 4) *Alistipes shahii* and *Odoribacter splanchnicus;* 5) *Butyricimonas virosa* and *Desulfovibrio piger;* 6) *Butyricimonas virosa* and *Alistipes putredinis;* 7) *Faecalibacterium prausnitzii* and *Phocaeicola plebeius;* 8) *Faecalibacterium prausnitzii* and *Oscillospiraceae bacterium;* 9) *Oscillospiraceae bacterium* and *Alistipes onderdonkii;* and 10) *Faecalibacterium prausnitzii* and *Alistipes onderdonkii.* The pairwise combinations in the present application are not limited to the examples listed above, and any two of the aforementioned species may be combined. There can be 45 pairwise combinations in total from the gut microbiota species provided in the present application.

In some specific embodiments, combinations of gut microbiota species (three-species combinations) may include the following: 1) *Alistipes shahii, Butyricimonas virosa,* and *Desulfovibrio piger;* 2) *Alistipes shahii, Butyricimonas virosa,* and *Alistipes putredinis;* 3) *Alistipes shahii, Butyricimonas virosa,* and *Odoribacter splanchnicus;* 4) *Butyricimonas virosa, Desulfovibrio piger,* and *Alistipes putredinis;* 5) *Butyricimonas virosa, Desulfovibrio piger,* and *Odoribacter splanchnicus;* 6) *Desulfovibrio piger, Alistipes putredinis,* and *Odoribacter splanchnicus;* 7) *Faecalibacterium prausnitzii, Phocaeicola plebeius,* and *Eubacterium rectale;* 8) *Phocaeicola plebeius, Faecalibacterium prausnitzii,* and *Oscillospiraceae bacterium;* 9) *Faecalibacterium prausnitzii, Oscillospiraceae bacterium,* and *Alistipes onderdonkii;* and 10) *Eubacterium rectale, Oscillospiraceae bacterium,* and *Alistipes onderdonkii.* The three-species combinations in the present application are not limited to the examples listed above, and any three of the aforementioned species may be combined. There can be 120 three-species combinations in total from the gut microbiota species provided in the present application.

In some specific embodiments, gut microbiota species combinations are not limited to the examples listed above, and the gut microbiota species may be combined in any other ways, including combinations of four, five, six, seven, eight, nine, or ten gut microbiota species.

In some specific embodiments, the gut microbiota includes at least one (such as one, two, three, four, five, at least one, at least two, at least three, at least four, or at least five) selected from the group consisting of *Alistipes shahii, Butyricimonas virosa, Desulfovibrio piger, Alistipes putredinis,* and *Odoribacter splanchnicus.*

The present application further provides a use of a composition in preparation of a drug for immunotherapy of CRC. The composition includes (1) at least one selected from the group consisting of (a) a gut microbiota or a metabolite thereof and (b) an antibody or a derivative thereof, and (2) a product for treating CRC;
the gut microbiota includes at least one selected from the group consisting of *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii;*
the antibody includes a monoclonal antibody targeting an IL-17A signaling pathway, a monoclonal antibody targeting CRC, or a combination thereof.

In some embodiments, the product for treating CRC includes at least one selected from the group consisting of a microbial strain, an immune checkpoint inhibitor, an immune activator, a cancer vaccine, an immune cell therapy drug, and a cytokine therapy drug.

In some embodiments, the microbial strain includes at least one selected from the group consisting of *Lactobacillus rhamnosus, Lactobacillus reuteri, Lactobacillus johnsonii,* and *Bifidobacterium.*

The gut microbiota or the metabolite thereof and/or the antibody or the derivative thereof in the present application may be used in combination with a microbial strain for treating CRC; or the gut microbiota or the metabolite thereof and/or the antibody or the derivative thereof in the present application may also be used in combination with the product (such as a drug) for treating CRC; thereby enhancing the sensitivity of a patient to a CRC immunotherapy drug and improving the therapeutic efficacy for CRC.

The microbial strain for treating CRC is a common strain for treating CRC in the art, such as *Lactobacillus rhamnosus, Lactobacillus reuteri, Lactobacillus johnsonii,* and *Bifidobacterium,* but the microbial strain is not limited to the aforementioned species.

The product for treating CRC is not limited to the products exemplified in the present application, and further includes products for treating CRC in the art, which all fall within the protection scope of the present application.

In the present application, compared with the use of a CRC immunotherapy drug alone, the combined use of the gut microbiota or the metabolite thereof and/or the antibody or the derivative thereof in the present disclosure with the CRC immunotherapy drug can significantly inhibit the growth of tumors (CRC).

In the present application, at least one selected from the group consisting of *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii* can also be used in combination with a microbial strain for treating CRC and a product for immunotherapy of CRC to enhance the therapeutic efficacy against CRC.

The present application also provides a use of at least one selected from the group consisting of (a) a gut microbiota or a metabolite thereof and (b) an antibody or a derivative thereof in preparation of a product for improving or predicting sensitivity of CRC to immunotherapy, where the immunotherapy comprising administering an immune checkpoint inhibitor to a subject in need; the gut microbiota includes at least one selected from the group consisting of *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii;*
the antibody includes a monoclonal antibody targeting an IL-17A signaling pathway, a monoclonal antibody targeting CRC, or a combination thereof.

In some embodiments, the metabolite of the gut microbiota includes at least one selected from the group consisting of O-phosphorylethanolamine, 5,6-dihydroxyindole-2-carboxylic acid, phosphatidylcholine (O-16:0_20:4), and 4-acetamidobutyric acid.

The gut microbiota or the metabolite thereof and/or the antibody or the derivative thereof in the present application can effectively inhibit the IL-17A signaling pathway, ameliorate the tumor microenvironment, enhance the anti-tumor effect of immunotherapy, and demonstrate the functions of regulating the immune microenvironment and improving the sensitivity to tumor immunotherapy.

The metabolites listed above in the present application, when used either alone or in combination, demonstrate significant anti-tumor effects.

In some embodiments, a metabolite of the *Alistipes shahii* includes the O-phosphorylethanolamine, the 5,6-dihydroxyindole-2-carboxylic acid, and the phosphatidylcholine (O-16:0_20:4).

In some embodiments, a metabolite of the *Butyricimonas virosa* includes the 4-acetamidobutyric acid.

In the present application, metabolites derived from the gut microbiota are also screened. O-phosphorylethanolamine, 5,6-dihydroxyindole-2-carboxylic acid, and phosphatidylcholine (O-16:0_20:4) are significantly enriched in *Alistipes shahii.* 4-acetamidobutyric acid is significantly enriched in *Butyricimonas virosa.*

In some specific embodiments, O-phosphorylethanolamine and 5,6-dihydroxyindole-2-carboxylic acid suppress the differentiation of naive CD4⁺ T cells into Th17 cells to reduce IL-17A secreted by Th17 cells, thereby down-regulating the activity of the IL-17A signaling pathway. Based on these findings, *Alistipes shahii* effectively suppresses the IL-17A signaling pathway through its metabolites O-phosphorylethanolamine and 5,6-dihydroxyindole-2-carboxylic acid, thereby ameliorating the tumor microenvironment and enhancing the anti-tumor efficacy of immunotherapy. This mechanism further elucidates the significant role of the metabolite of the gut microbiota in modulating the immune microenvironment and improving the sensitivity to tumor immunotherapy.

According to experiments, these metabolites all can significantly reduce the infiltration of Th17 cells and exhausted immune cells (PD-1⁺ cells) in the tumor, and enhance the infiltration and functionality of CD8⁺ T cells in the tumor.

The present application also provides a use of at least one selected from the group consisting of (a) a gut microbiota or a metabolite thereof and (b) an antibody or a derivative thereof in preparation of a drug for inhibiting an IL-17A signaling pathway; the gut microbiota includes at least one selected from the group consisting of *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii;*
the metabolite includes any one or a combination of two or more selected from the group consisting of O-phosphorylethanolamine, 5,6-dihydroxyindole-2-carboxylic acid, phosphatidylcholine (O-16:0_20:4), and 4-acetamidobutyric acid;
the antibody includes a monoclonal antibody targeting the IL-17A signaling pathway, a monoclonal antibody targeting CRC, or a combination thereof.

In the present application, bulk transcriptomes from tumor tissues of MPR patients are subjected to KEGG enrichment analysis and GSEA. According to analysis results, the IL-17 signaling pathway is significantly down-regulated in pre-treatment tumor tissues of the MPR patients compared to non-MPR patients. IL-17 signaling pathway and Th17 cell differentiation pathway are significantly down-regulated in pre-treatment tumor tissues of the MPR patients compared to the non-MPR patients. IL-17A signaling pathway and Th17 cell differentiation pathway are significantly down-regulated in post-treatment tumor tissues of the MPR patients compared to the non-MPR patients.

According to experimental results, the gut microbiota or the metabolite thereof and/or the antibody or the derivative thereof in the present application can suppress the secretion of IL-17 by inhibiting the differentiation of naive CD4⁺ T cells into Th17 cells, and ameliorate the immunosuppressive status in the tumor microenvironment, thereby enhancing the sensitivity of MSS CRC patients to immunotherapy and facilitating an anti-tumor immune response.

The present application also provides a use of a monoclonal antibody targeting an IL-17A signaling pathway in combination with an immune checkpoint inhibitor in preparation of a drug for treating CRC.

The present application has further verified that the combined use of the monoclonal antibody targeting the IL-17A signaling pathway with the immune checkpoint inhibitor (including, but not limited to, an anti-PD-1 monoclonal antibody) demonstrates a significant tumor-suppressive effect.

In some embodiments, the immune checkpoint inhibitor includes an anti-PD-1 antibody or an anti-PD-L1 antibody.

In some embodiments, the immune checkpoint inhibitor is an anti-PD-1 antibody.

The immune checkpoint inhibitor in the present application is not limited to the anti-PD-1 antibody or the anti-PD-L1 antibody, and further includes immune checkpoint inhibitors commonly adopted in the art.

The gut microbiota or the metabolite thereof and/or the antibody or the derivative thereof in the present application can enhance the sensitivity of CRC patients to an anti-PD-1 antibody therapy. Compared with anti-PD-1 antibody monotherapy, the combined use of the gut microbiota or the metabolite thereof and/or the antibody or the derivative thereof with the anti-PD-1 antibody can significantly inhibit the growth of CRC.

In some embodiments, the CRC includes MSS CRC and MSI CRC.

In some specific embodiments, the CRC includes MSI-H CRC.

The term "MSS CRC" as used herein encompasses well-recognized pathological types of MSS CRC in the art, including, for example, MSS CRC in situ.

In some embodiments, a dosage form of the drug includes one selected from the group consisting of liquid, gas, solid, and semi-solid dosage forms. The dosage form of the drug involved in the present application is not limited to the aforementioned dosage forms, and also includes common dosage forms in the art. The gut microbiota or the metabolite thereof and/or the antibody or the derivative thereof in the present application can be prepared into a pharmaceutically acceptable product of any dosage form, such as a capsule, a powder, a granule, a tablet, a drop pill, an oral liquid, a lyophilized powder injection, or an injectable solution. During the preparation of the product, an appropriate pharmaceutical excipient may be added as needed. The pharmaceutical excipient may include any one pharmaceutically acceptable excipient or a combination of any two or more pharmaceutically acceptable excipients.

In some embodiments, the drug may include a pharmaceutically acceptable excipient.

In some specific embodiments, the pharmaceutically acceptable excipient includes at least one selected from the group consisting of a solubilizing agent, a cosolvent, a preservative, a flavoring agent, a coloring agent, a suspending agent, an emulsifying agent, a wetting agent, a foaming agent, a defoaming agent, a filler, an absorbent, a diluent, and a binder. In the present application, the pharmaceutically acceptable excipient can be selected according to actual conditions, and is not limited to the aforementioned categories.

The present application further provides a pharmaceutical composition including (1) a gut microbiota or a metabolite thereof, and/or an antibody or a derivative thereof, and (2) a drug for immunotherapy of CRC;
the gut microbiota includes at least one selected from the group consisting of *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii;*
the antibody includes a monoclonal antibody targeting an IL-17A signaling pathway, a monoclonal antibody targeting CRC, or a combination thereof.

In some embodiments, the drug for immunotherapy of CRC includes an immune checkpoint inhibitor.

In some embodiments, the immune checkpoint inhibitor includes an anti-PD-1 antibody or an anti-PD-L1 antibody.

In some embodiments, the gut microbiota further includes a microbial strain for treating CRC.

In some embodiments, the microbial strain for treating CRC includes at least one selected from the group consisting of *Lactobacillus rhamnosus, Lactobacillus reuteri, Lactobacillus johnsonii,* and *Bifidobacterium.*

The pharmaceutical composition combining (1) the gut microbiota or the metabolite thereof, and/or the antibody or the derivative thereof, and (2) the drug for immunotherapy of CRC in the present application can significantly inhibit the growth of a tumor, particularly the growth of CRC. Moreover, the gut microbiota or the metabolite thereof and/or the antibody or the derivative thereof utilized in the present application can enhance the sensitivity of patients to CRC immunotherapy drugs, thereby further improving the therapeutic efficacy against CRC.

In some specific embodiments, the pharmaceutical composition of the present application can be prepared into an oral preparation or a dietary supplement, which can be conveniently administered. In contrast, some of the existing immune enhancement therapies, such as cell therapy and gene therapy, require complex production processes and high costs, which limits the widespread application. The gut microbiota employed in the present application can be produced on a large scale through mature fermentation and formulation processes. These processes offer advantages such as low cost and short production cycle, thereby facilitating promotion and popularization.

In some embodiments, the gut microbiota is mixed with a solvent to produce a microbial agent. A mass concentration of the gut microbiota in the microbial agent is 10⁸ colony-forming unit (CFU) per dose to 10¹² CFU per dose.

In some embodiments, the solvent includes PBS. The solvent adopted in the present application is not limited to PBS, and any solvent for preparing the microbial agent falls within the scope of the present application.

The present application further provides a method for enhancing sensitivity of CRC to an immunotherapy drug, including administering (1) at least one selected from the group consisting of (a) a gut microbiota or a metabolite thereof and (b) an antibody or a derivative thereof, and (2) an immune checkpoint inhibitor;
the gut microbiota includes at least one selected from the group consisting of *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii;*
the antibody includes a monoclonal antibody targeting an IL-17A signaling pathway, a monoclonal antibody targeting CRC, or a combination thereof.

In some embodiments, the method further includes administering an immune cell therapy drug and a cytokine therapy drug.

The existing immunotherapy strategies, such as PD-1 or PD-L1 inhibitors, often demonstrate limited efficacy against MSS CRC, and many patients exhibit poor therapeutic responses to these immunotherapy strategies. Current strategies for improving the sensitivity to immunotherapy may involve complex gene editing, targeted drugs, or chemotherapy, which are invasive and accompanied by significant side effects.

In some embodiments, the derivative in the term "an antibody or a derivative thereof" includes, but is not limited to, an antigen-binding fragment and a related antibody-drug conjugate (ADC). In some embodiments, the antigen-binding fragment includes, but is not limited to: Fab, Fab', F(ab')₂, ScFv, BiTE, a single-domain antibody (sdAb) (such as a camelid/alpaca antibody VHH, a shark antibody VNAR, and humanized VH/VL), a diabody, a minibody, a bispecific antibody fragment BiTE, a triabody, and a VHH-based nanobody (such as Nanobody).

The gut microbiota or the metabolite thereof and/or the antibody or the derivative thereof provided in the present application can modulate the gut microbiota and immune system to enhance the sensitivity of CRC patients to immunotherapy, particularly the sensitivity of MSS CRC patients to immunotherapy, such that increased patients can benefit from the immunotherapy. The present application employs safe biological agents, which do not require complex surgical procedures or pharmaceutical interventions, demonstrate favorable tolerance and low side effects, and are suitable for long-term use.

Compared with the prior art, the present application has the following beneficial effects:

The present application provides a use of a gut microbiota or a metabolite thereof and/or an antibody or a derivative thereof in preparation of a drug for immunotherapy of CRC. The gut microbiota or the metabolite thereof and/or the antibody or the derivative thereof in the present application can ameliorate the tumor microenvironment, enhance the anti-tumor efficacy of immunotherapy. Further, the gut microbiota in the present application can be produced on a large scale through mature fermentation and formulation processes. These processes offer advantages such as low cost and short production cycle, thereby facilitating promotion and popularization. Additionally, the gut microbiota or the metabolite thereof in the present application can predict the survival, prognosis, and immunotherapy sensitivity in MSS CRC patients.

In the present disclosure, the technical features described in an open-ended manner include a closed technical solution composed of the listed features, as well as an open-ended technical solution including the listed features.

In the present disclosure, unless otherwise specified, a numerical range referred to is deemed continuous, and includes minimum and maximum values of the range and any value between the minimum and maximum values. Further, when the range refers to integers, any integer between the minimum and maximum values of the range is included. In addition, when a plurality of ranges are provided to describe a feature or characteristic, the ranges can be combined. In other words, unless otherwise indicated, all ranges disclosed herein shall be understood to include any and all sub-ranges belonging to the ranges.

In the present disclosure, the term "subject" should be interpreted in the broadest sense, and refers to any individual administered with the substance or subjected to the method. This term includes, but is not limited to: an individual diagnosed with CRC, an individual at risk of developing CRC, an individual at any stage or phase of CRC, an individual administered with the substance or subjected to the method for a therapeutic, preventive, or adjuvant therapeutic purpose, and an individual administered with the substance or subjected to the method for scientific research or a clinical trial. It should be understood that the scope of the term "subject" shall encompass all individuals to whom the substance is administered or on whom the method is performed in the context of medical, veterinary, or research practices. The subject includes both a human subject and a non-human subject. The non-human subject includes, but is not limited to: mammals (such as dogs, cats, horses, cattle, sheep, goats, pigs, rabbits, and rodents (such as mice and rats)), birds, fish, and any other animals that may benefit from the substance or the therapeutic method.

To better illustrate the objective, technical solutions, and advantages of the present application, the present application will be further explained below with reference to the accompanying drawings and specific embodiments. In the following embodiments, unless otherwise specified, the experimental methods used are conventional, the materials and reagents used are commercially available, and component materials used in parallel experiments are of a same type.

### Example 1 Screening of gut microbiota through metagenomic sequencing of fecal samples

In the present application, 17 pre-treatment fecal samples were collected from sensitive (MPR) patients and 8 pre-treatment fecal samples were collected from insensitive (non-MPR) patients. All samples were each subjected to metagenomic sequencing analysis.

### Detailed steps for metagenomic sequencing:

### 1. Test method:

Total DNA was isolated from each sample and purified using a magnetic bead-based fecal genomic DNA extraction kit (pre-packaged) (AU46111-96, Bioteke) according to a protocol provided by the manufacturer. A DNA concentration was then detected using Qubit 1X dsDNA HS Assay Kit (invitrogen, Q33230). 200 ng of DNA meeting quality control (QC) requirements was taken and transferred to a 0.6 mL low-adsorption microcentrifuge tube (#MCT-060-L-C), and water was added to a total volume of 52 µL. The DNA was then fragmented using a fragmentation device (Bioruptor^{™} Pico, Diagenode) with parameters adjusted according to a required fragment length (generally 200 bp to 500 bp). Fragmented products were purified and recovered using magnetic beads from a TruSeq library preparation kit. A size of the fragmented products was verified using an Agilent 2100 Bioanalyzer (HighSensitivity DNA Reagents, Agilent, 5067-4627). If the fragmented products failed to meet the required fragment length range, re-sampling and fragmentation were required. The fragmented products were used to construct a genomic library with TruSeq Nano DNA LT Library Preparation Kit (Illumina, FC-121-4001). The genomic library was constructed through end repair, adapter ligation, index PCR amplification, and purification, with detailed procedures following instructions of the kit. The constructed library was quantified using Qubit 1X dsDNA HS Assay Kit (invitrogen, Q33230). Finally, paired-end sequencing in a sequencing mode PE150 was conducted on Illumina Novaseq 6000 (LC Bio Technology CO., Ltd. Hangzhou, China) with NovaSeq 6000 XP 4-Lane Kit v1.5 (300 cycles) (Illumina, 20043131) according to standard operations.

### 2. Bioinformatic analysis method:

Raw sequencing data was output in a fastq format, and was subjected to quality control using fastp (https://github.com/OpenGene/fastp, version 0.23.4) software with parameters: -1 100 -g -W 6 -5 -q 20 -u 30. The sequencing data was aligned to a host genome using bowtie2 (https://www.sbgrid.org/software/titles/bowtie-2, version 2.2.0). Sequences mapping to the host genome were filtered out (this step was applied merely to projects requiring host sequence removal, such as human or mouse fecal samples), so as to ensure that all subsequent assembly and analysis results were microbial sequences. After clean reads were acquired, clean reads for each sample were assembled using software MEGAHIT (https://github.com/voutcn/megahit, version 1.2.9). Assembly results were output in a FASTA format, where each sequence represents a contig. In the present application, contig sequences with a length of greater than 500 bp were retained, and coding sequences (CDSs) were predicted using software MetaGeneMark (https://exon.gatech.edu/GeneMark/meta_gmhmmp.cgi, version 3.26). Based on prediction results, CDSs with a length of less than 100 nt were filtered out. According to CDS prediction results, deduplication was performed with MMseqs2 software (https://github.com/soedinglab/MMseqs2/, version 15-6f452) to produce non-redundant Unigenes. Clustering was performed with an identity of 95% and a coverage of 90%. With the longest sequence as a representative sequence, a Unigene set was constructed. Clean reads for each sample were aligned to each Unigene sequence using Bowtie2 (https://www.sbgrid.org/software/titles/bowtie-2, version 2.2.0). The number of reads for each sample that mapped to each Unigene was calculated. Unigenes to which 2 or less reads were mapped across all samples were filtered out to obtain a final Unigene set for subsequent analysis. Abundance of each retained Unigene was calculated. Protein sequences of Unigenes were aligned to the NR_meta database using DIAMOND software (https://github.com/bbuchfink/diamond, version 0.9.14) to obtain taxonomic annotations at different classification levels, and were also aligned to functional databases including KEGG (http://www.genome.jp/kegg/, version 87.1), GO (http://geneontology.org/, version 2018.12.21), eggNOG (http://eggnogdb.embl.de/download/, version 5.0), PHI (http://www.phi-base.org/, version 4.14), CAZy (http://www.cazy.org/, version 2022.08.06), CARD (https://card.mcmaster.ca/download, version v3.2.5), VFDB (http://www.mgc.ac.cn/VFs/, version 2023.03.03), MGEs (http://github.com/KatariinaParnanen/MobileGeneticElementDatabase, version 2017.12.28), and BacMet (http://bacmet.biomedicine.gu.se/, version 2.0) (details were shown in Report 2.3) to obtain annotations corresponding to each functional database. Finally, abundance information for each taxonomic and functional classification level was acquired through Unigene abundance statistics. Differential statistical analysis was performed between comparison groups based on taxonomy and functionality. Specifically, Fisher's exact test was adopted for differential comparison of samples without biological replicates, Wilcoxon rank-sum test (also known as Mann Whitney U test) was adopted for differential comparison between two groups of samples with biological replicates, and Kruskal-Wallis test was adopted for comparison among a plurality of groups of samples with biological replicates. A differential threshold was defined as p < 0.05 and |log2(fold_change)| > 1. Based on differential analysis results, a grouped box plot was generated using R language (version 3.6.0) to show the abundance of differential species or differential functional entries across groups. Functional enrichment analysis was performed using ReporterScore (https://github.com/Asa12138/ReporterScore). Alpha diversity indices, including Chao1, Observed species, Goods coverage, Shannon, and Simpson were calculated at a species level using QIIME1 (http://qiime.org/), and visualized as rarefaction curves (R language, version 3.6.0). For samples with five or more biological replicates, intergroup differential analysis was performed. Results were displayed using violin and box plots. Wilcoxon rank-sum test (also known as Mann Whitney U test) was adopted for two-group comparison, and Kruskal-Wallis test was adopted for multi-group comparison. Beta diversity was assessed by calculating Bray-Curtis dissimilarity and visualized through principal coordinates analysis (PCoA), nonmetric multidimensional scaling (NMDS), unweighted pair group method with arithmetic mean (UPGMA), analysis of similarities (ANOSIM), and permutational multivariate analysis of variance (Adonis, PermANOVA) (R language, version 3.6.0).

### 3. Species screening:

Linear discriminant analysis effect size (LEfSe) analysis was conducted to identify intergroup differences in species across taxonomic levels. A screening threshold was defined as linear discriminant analysis (LDA) > 2.5 and p < 0.05. Results were visualized through cladograms and bar charts. Spearman's correlation analysis was performed at a species level using R packages ggplot2 (version 3.2.0) and ggnetwork, and correlation pairs of |rho| > 0.8 were visualized through a network graph. An R package metagenomeSeq (version 1.38.0) was adopted for differential analysis of species between two groups at a species level, and results were visualized by a Manhattan plot (ggplot2, version 3.2.0).

### 4. Analysis results:

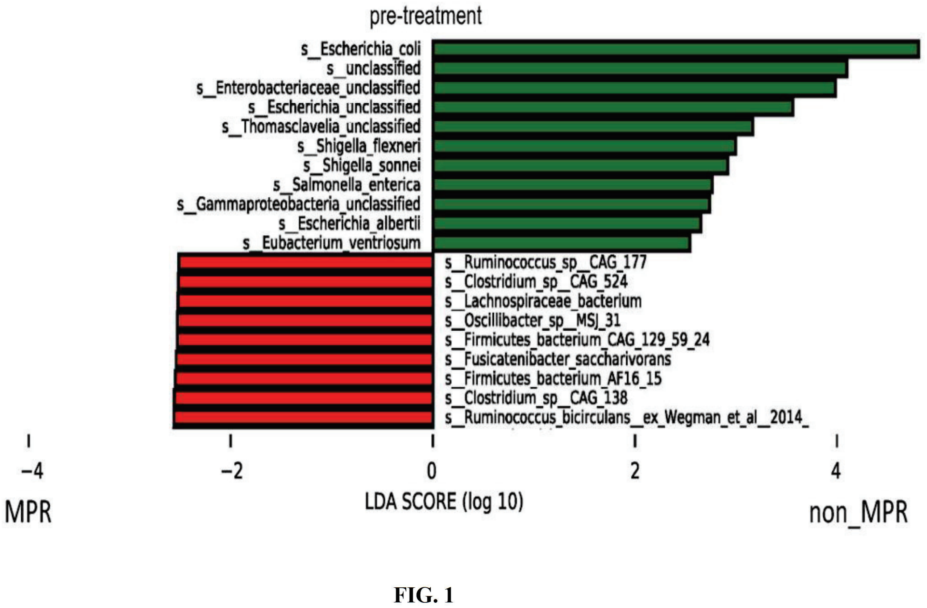

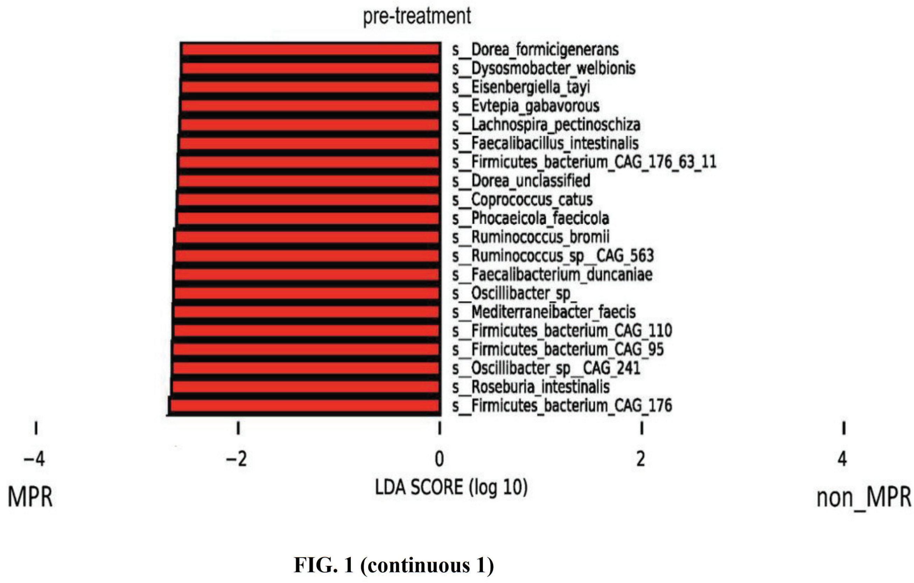

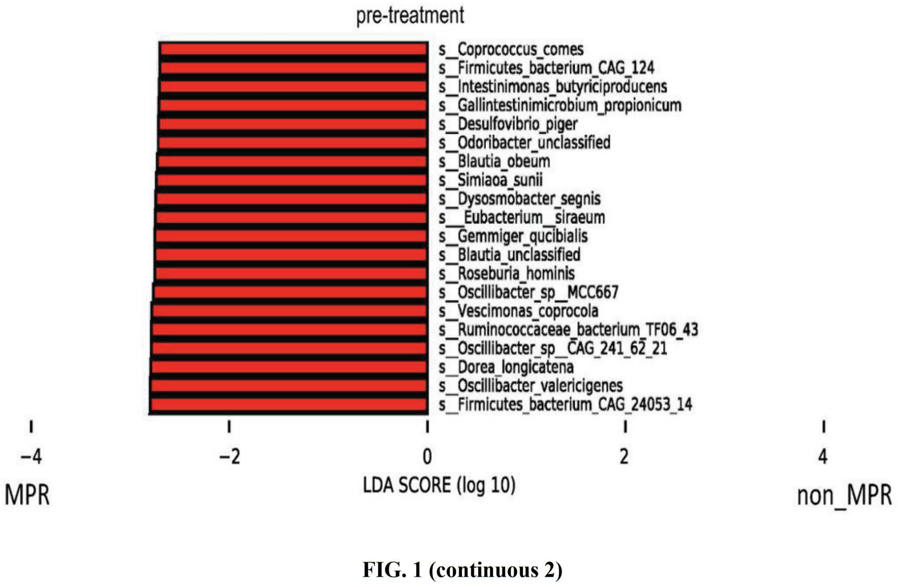

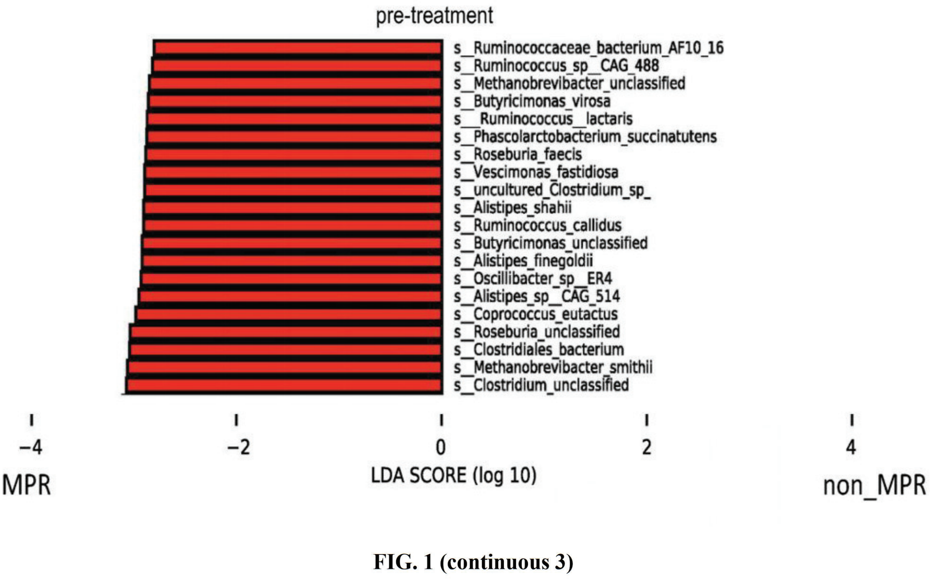

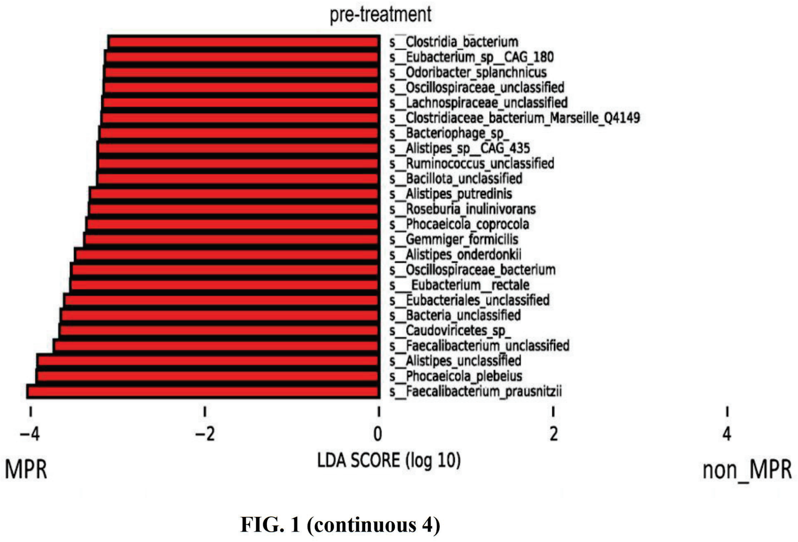

As shown in FIG. 1, together with FIG. 1 (continuous 1), FIG. 1 (continuous 2), FIG. 1 (continuous 3), and FIG. 1 (continuous 4), the comparison of the pre-treatment samples from the sensitive (MPR) patients with the pre-treatment samples from the insensitive (non-MPR) patients showed that 78 species were significantly enriched in the sensitive patients. In the present application, the following ten bacterial species were selected for subsequent research: *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii.*

Details are as follows:
*Alistipes shahii:* abundance ranking: 23, and linear discriminant analysis (LDA) score ranking: 24;
*Butyricimonas virosa:* abundance ranking: 25, and LDA score ranking: 30;
*Desulfovibrio piger:* abundance ranking: 48, and LDA score ranking: 46;
*Alistipes putredinis:* abundance ranking: 11, and LDA score ranking: 10;
*Odoribacter splanchnicus:* abundance ranking: 13, and LDA score ranking: 14;
*Faecalibacterium prausnitzii:* abundance ranking: 1, and LDA score ranking: 1;
*Phocaeicola plebeius:* abundance ranking: 2, and LDA score ranking: 2;
*Eubacterium rectale:* abundance ranking: 5, and LDA score ranking: 4;
*Oscillospiraceae bacterium:* abundance ranking: 6, and LDA score ranking: 5; and
*Alistipes onderdonkii:* abundance ranking: 8, and LDA score ranking: 6.

### Example 2 Gut microbiota validation experiment

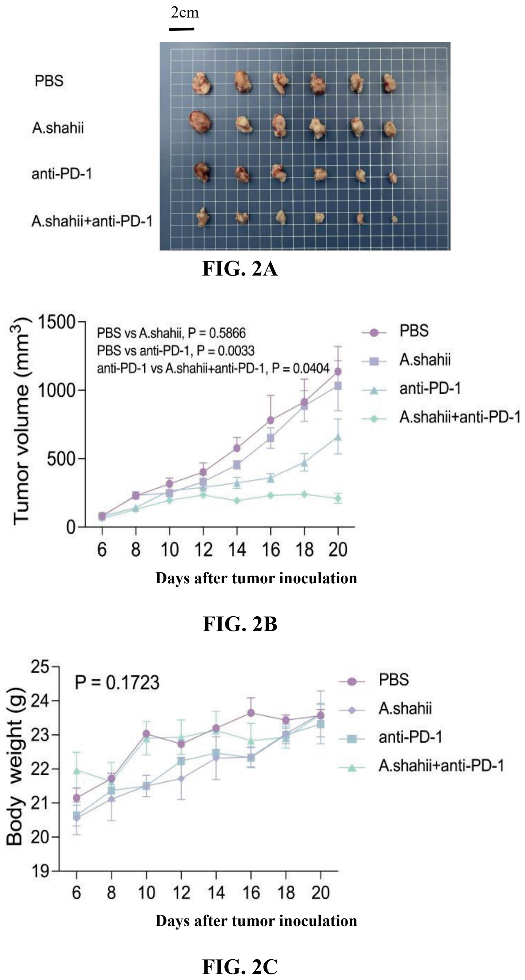

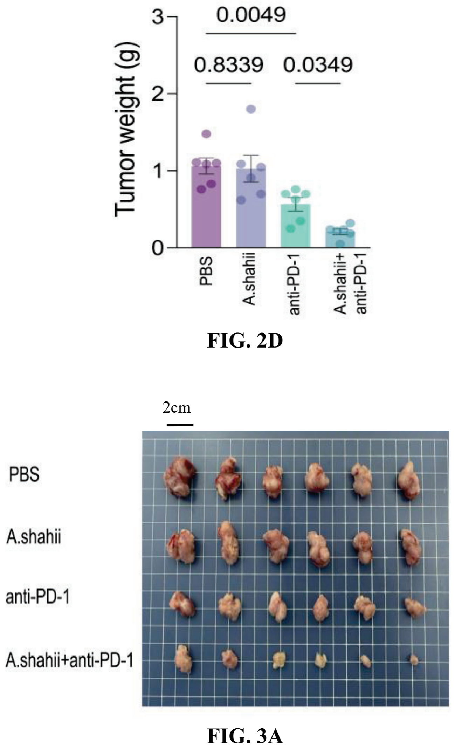

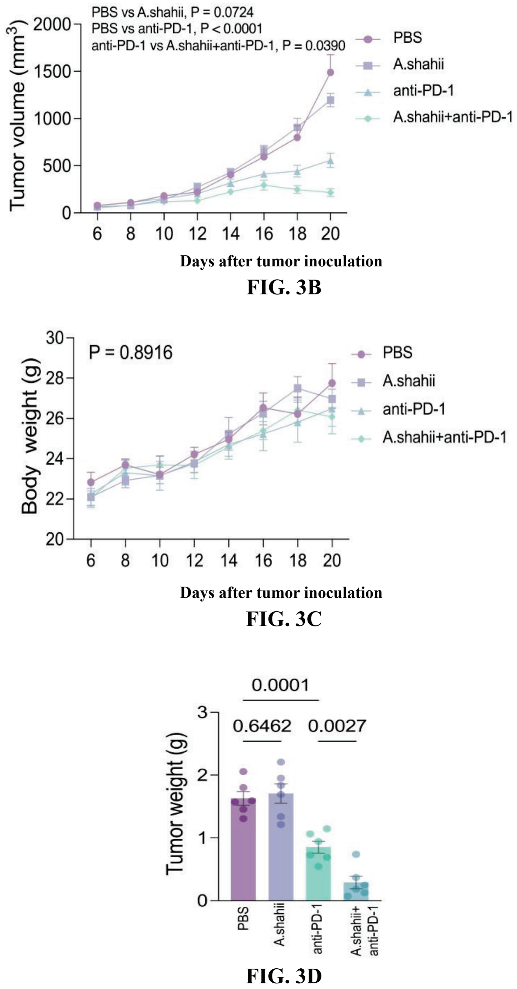

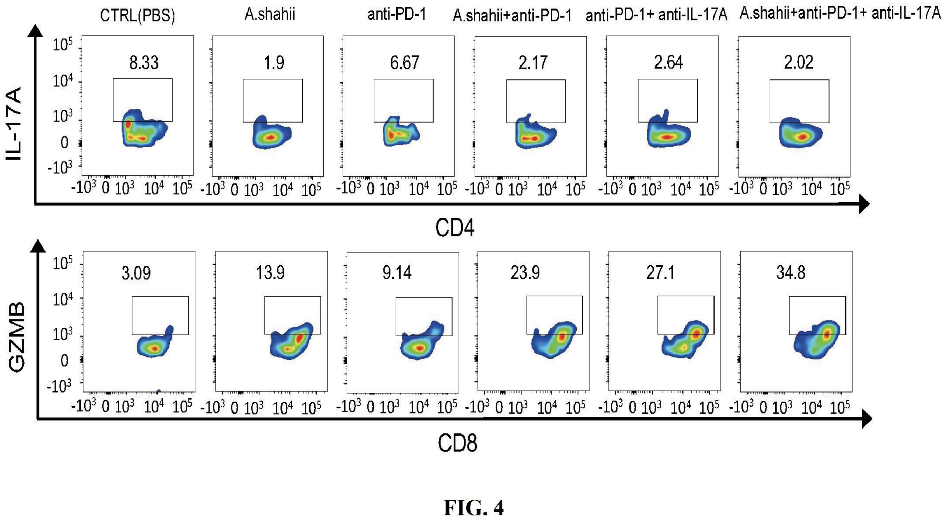

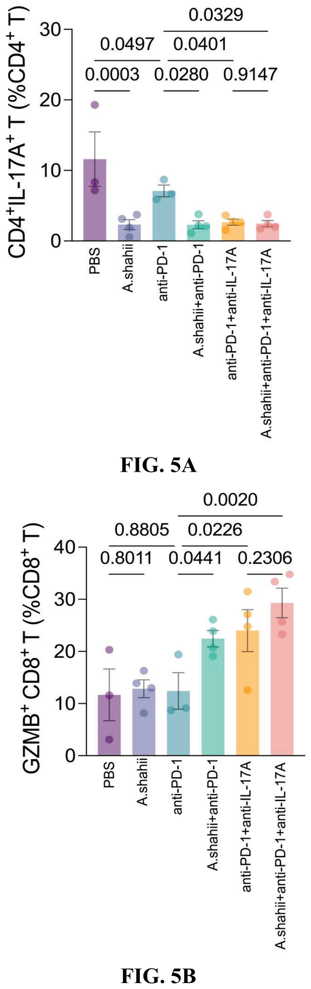

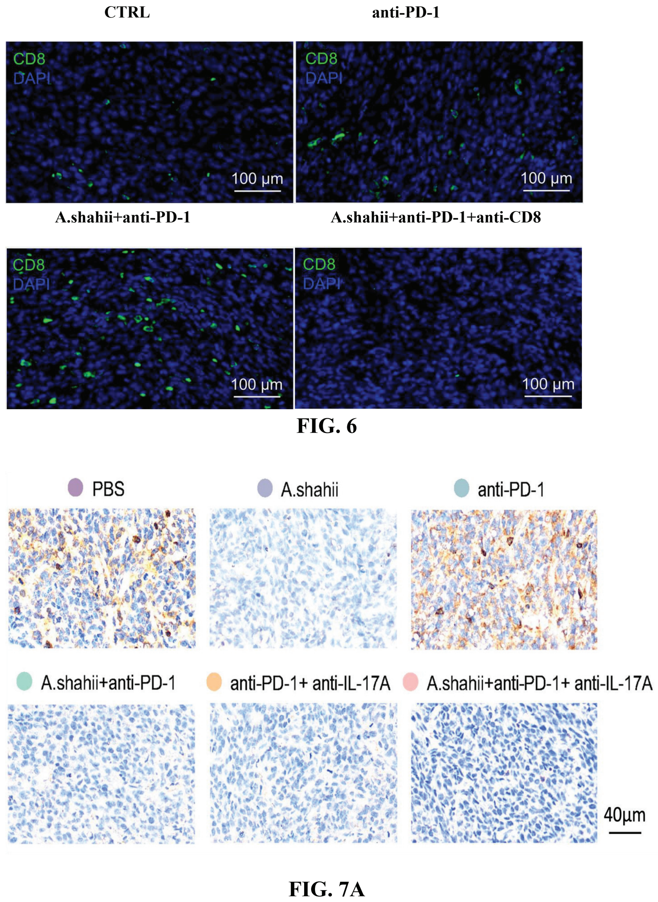

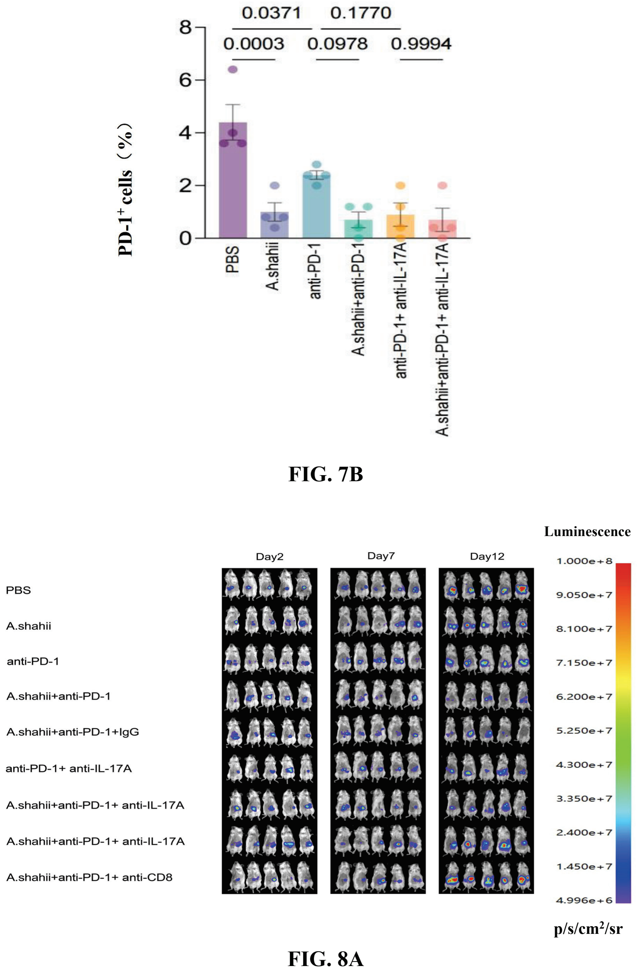

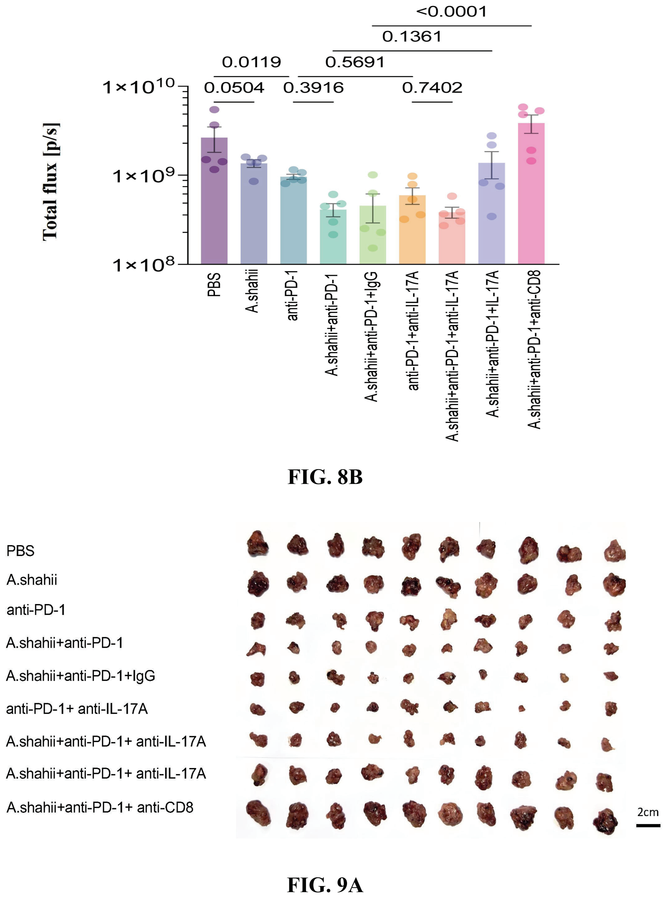

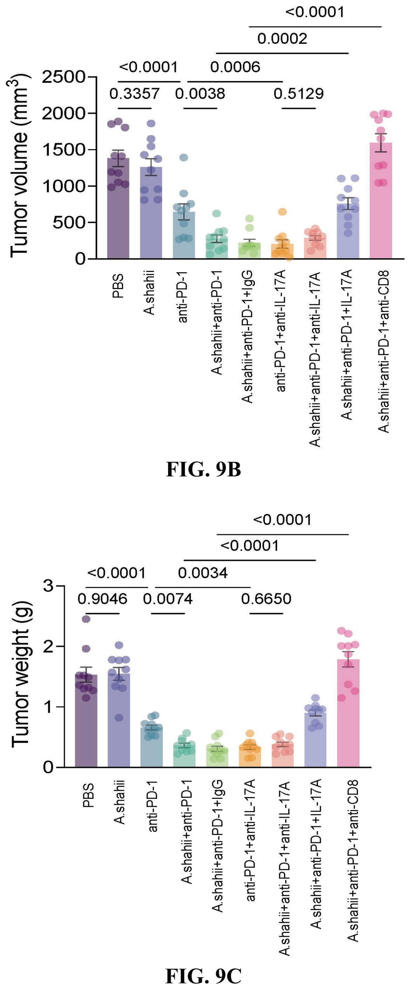

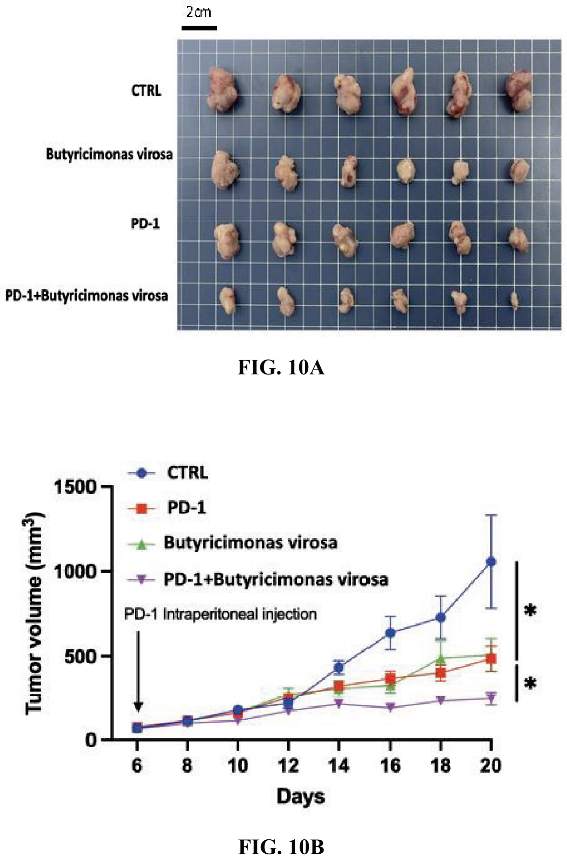

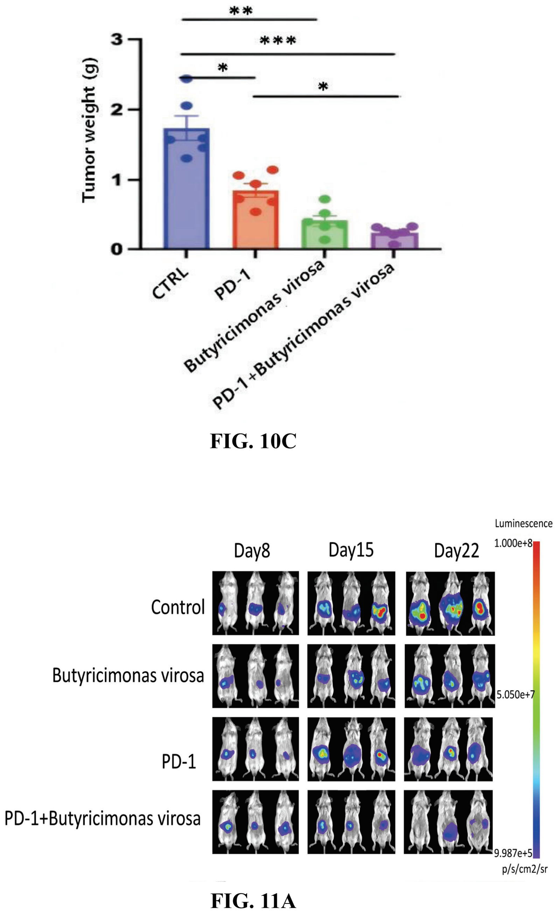

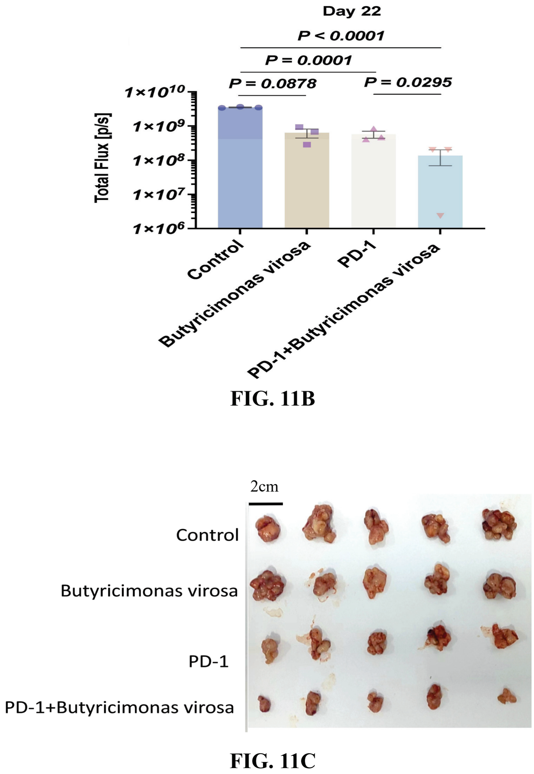

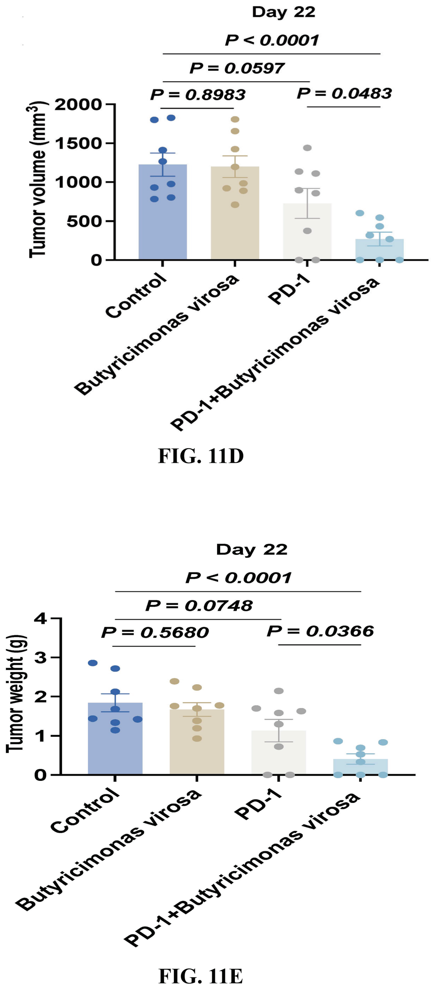

1. Sources of gut microbiota: The gut microbiota were purchased through the official channel of the American Type Culture Collection (ATCC) in a form of a lyophilized powder, and were stored at 2°C to 8°C, including:
   *Alistipes shahii* (ATCC No.: BAA-1179, Batch No.: 70061103);
   *Desulfovibrio piger* (ATCC No.: 29098, Batch No.: 70037201);
   *Alistipes putredinis* (ATCC No.: 29800, Batch No.: 70051086); and
   *Odoribacter splanchnicus* (ATCC No.: 29572, Batch No.: 70054363).
   *Butyricimonas virosa* was supplied by Beijing Biobw Biotechnology Co., Ltd. in a form of a lyophilized powder, and was stored at 2°C to 8°C.
2. Culture conditions:
   *Alistipes shahii, Alistipes putredinis,* and *Odoribacter splanchnicus* were cultured with ATCC medium 1490 at 37°C in an anaerobic environment (including 80% of N₂, 10% of CO₂, and 10% of H₂).
   *Desulfovibrio piger* was cultured with ATCC medium 1249 at 37°C in an anaerobic environment (including 80% of N₂, 10% of CO₂, and 10% of H₂).
   *Butyricimonas virosa* was cultured with a nutrient broth medium from Solarbio at 37°C in an anaerobic environment (including 80% of N₂, 10% of CO₂, and 10% of H₂).
3. Steps for primary recovery:
   (1) Recovery of *Alistipes shahii, Alistipes putredinis,* and *Odoribacter splanchnicus:*
      1) Under anaerobic conditions, a lyophilized powder was dissolved in approximately 0.5 mL of pre-deoxygenated sterile ATCC medium 1490 to produce a solution.
      2) Under aseptic conditions, the solution was transferred to 5-6 mL of sterile ATCC medium 1490 to produce a primary broth.
      3) Several drops were taken from the primary broth and inoculated onto either an ATCC medium 260 plate or an ATCC medium 260 agar slant.
      4) Incubation was conducted in a 37°C anaerobic incubator for 2 days to 4 days.
   (2) Recovery of *Desulfovibrio piger:*
      1) Under anaerobic conditions, a lyophilized powder was dissolved in approximately 0.5 mL of pre-deoxygenated sterile ATCC medium 1249 to produce a solution.
      2) Under aseptic conditions, the solution was transferred to 5-6 mL of sterile ATCC medium 1249 to produce a primary broth.
      3) Several drops were taken from the primary broth and inoculated onto either an ATCC medium 260 plate or an ATCC medium 260 agar slant.
      4) Incubation was conducted in a 37°C anaerobic incubator for 2 days to 4 days.
   (3) Recovery of *Butyricimonas virosa:*
      1) 0.3 mL of a pre-deoxygenated medium (or sterile water) was pipetted by a sterile pipette and added to a lyophilized strain, and shaking was conducted gently until complete dissolution to produce a solution.
      2) The solution was completely transferred to a liquid medium or inoculated onto no more than two plates (if the liquid medium was adopted, an anaerobic screw-cap test tube was employed, and oxygen was removed with high-purity nitrogen).
      3) An inoculated culture was immediately cultured in an anaerobic environment at 37°C for 2 days to 4 days.
4. Validation in subcutaneous CRC mouse models:
   (1) Model construction:
      Six-week-old C57BL/6 and BALB/c mice were each randomly divided into the following four groups:
      a CTRL group, an anti-PD-1 monoclonal antibody group (abbreviated as anti-PD-1 group or PD-1 group), an *Alistipes shahii* group, and an *Alistipes shahii* + anti-PD-1 group.
      Specific treatments for the groups were as follows:
         CTRL group: Mice were intragastrically administered with 200 µL of PBS every other day until an experimental endpoint.
         *Alistipes shahii* group: Mice were intragastrically administered with 1 × 10⁹ CFU of *Alistipes shahii* (resuspended in 200 µL of PBS) every other day until an experimental endpoint.
   (2) Cell injection and tumor model establishment:
      After four rounds of intragastric administration of bacteria or PBS, mice were subcutaneously injected with an MSI-H MC38 cell line (2,000,000/mouse) or an MSS CT26 cell line (2,000,000/mouse) to establish a mouse model of subcutaneous CRC.
      A tumor size and an animal body weight were monitored every other day.
      When a tumor volume reached 80 mm³, based on the *Alistipes shahii* group, an anti-PD-1 monoclonal antibody was intraperitoneally injected (100 µg/mouse, twice weekly) until an experimental endpoint to create the *Alistipes shahii* + anti-PD-1 group.
   (3) Experimental results: In both C57BL/6 mice (mouse models of subcutaneous MSI-H CRC) and BALB/c mice (mouse models of MSS CRC), *Alistipes shahii* enhanced the sensitivity of mice to the anti-PD-1 monoclonal antibody therapy. Compared to the anti-PD-1 monoclonal antibody monotherapy, the combination of *Alistipes shahii* with the anti-PD-1 monoclonal antibody could significantly inhibit the tumor growth (as shown in FIG. 2A, FIG. 2B, FIG. 2C, FIG. 2D, FIG. 3A, FIG. 3B, FIG. 3C, and FIG. 3D).
      Therefore, *Alistipes shahii* may suppress the infiltration of Th17 cells in the tumor, and promote the infiltration and functionality of CD8⁺ T cells in the tumor, thereby facilitating an anti-tumor immune response.
5. Validation in mouse models of orthotopic CRC:
   (1) Model construction:
      Six-week-old BALB/c mice were randomly divided into the following nine groups:
      a PBS group, an anti-PD-1 group, an *Alistipes shahii* group, an *Alistipes shahii* + anti-PD-1 group, an anti-PD-1 *+ Alistipes shahii* + immunoglobulin G (IgG) group, an anti-PD-1 + anti-IL-17A monoclonal antibody (abbreviated as anti-IL-17A) group, an anti-PD-1 *+ Alistipes shahii* + anti-IL-17A group, an anti-PD-1 *+ Alistipes shahii* + IL-17A group, and an anti-PD-1 *+ Alistipes shahii* + anti-CD8 monoclonal antibody (abbreviated as anti-CD8) group. Specific treatments for the groups were as follows:
   (2) Cell injection and tumor model establishment:
      1) One week after intragastric administration of PBS, on Day 0, an MSS CT26-luc cell line (500,000 cells/mouse) was injected into the cecal serosal layer of mice to establish a mouse model of orthotopic CRC. Mice were intragastrically administered with 200 µL of PBS every other day until an experimental endpoint to create the PBS group.
      2) On Day 0, an MSS CT26-luc cell line (500,000 cells/mouse) was injected into the cecal serosal layer of mice to establish a mouse model of orthotopic CRC. When a tumor volume reached 80 mm³, an anti-PD-1 monoclonal antibody was intraperitoneally injected (100 µg/mouse, twice weekly until an experimental endpoint) to create the anti-PD-1 group.
      3) One week after intragastric administration of *Alistipes shahii,* on Day 0, an MSS CT26-luc cell line (500,000 cells/mouse) was injected into the cecal serosal layer of mice to establish a mouse model of orthotopic CRC. Mice were intragastrically administered with 1 × 10⁹ CFU of *Alistipes shahii* (resuspended in 200 µL of PBS) every other day until an experimental endpoint to create the *Alistipes shahii* group.
      4) Based on the *Alistipes shahii* group, when a tumor volume reached 80 mm³, an anti-PD-1 monoclonal antibody was intraperitoneally injected (100 µg/mouse, twice weekly until an experimental endpoint) to create the *Alistipes shahii +* anti-PD-1 group.
      5) Based on the *Alistipes shahii* group, when a tumor volume reached 80 mm³, an anti-PD-1 monoclonal antibody (100 µg/mouse, twice weekly until an experimental endpoint) and IgG (200 µg/mouse, twice weekly until an experimental endpoint) were each intraperitoneally injected to create the anti-PD-1 *+ Alistipes shahii* + IgG group.
      6) On Day 0, an MSS CT26-luc cell line (500,000 cells/mouse) was injected into the cecal serosal layer of mice to establish a mouse model of orthotopic CRC. When a tumor volume reached 80 mm³, an anti-PD-1 monoclonal antibody (100 µg/mouse, twice weekly until an experimental endpoint) and an anti-IL-17A monoclonal antibody (200 µg/mouse, twice weekly until an experimental endpoint) were each intraperitoneally injected to create the anti-PD-1 + anti-IL-17A group.
      7) Based on the *Alistipes shahii* group, when a tumor volume reached 80 mm³, an anti-PD-1 monoclonal antibody (100 µg/mouse, twice weekly until an experimental endpoint) was intraperitoneally injected, and IL-17A (0.5 µg/mouse, every other day until an experimental endpoint) was injected through a tail vein, to create the anti-PD-1 *+ Alistipes shahii* + IL-17A group.
      8) Based on the *Alistipes shahii* group, when a tumor volume reached 80 mm³, an anti-PD-1 monoclonal antibody (100 µg/mouse, twice weekly until an experimental endpoint) and an anti-IL-17A monoclonal antibody (200 µg/mouse, twice weekly until an experimental endpoint) were each intraperitoneally injected to create the anti-PD-1+ *Alistipes shahii +* anti-IL-17A group.
      9) Based on the *Alistipes shahii* group, when a tumor volume reached 80 mm³, an anti-PD-1 monoclonal antibody (100 µg/mouse, twice weekly until an experimental endpoint) and an anti-CD8 monoclonal antibody (100 µg/mouse, twice weekly until an experimental endpoint) were each intraperitoneally injected to create the anti-PD-1 *+ Alistipes shahii* + anti-CD8 group.
   (3) Experimental results:
      In BALB/c mice (models of orthotopic MSS CRC), *Alistipes shahii* could enhance the sensitivity of mice to the anti-PD-1 monoclonal antibody therapy. Compared to the anti-PD-1 monoclonal antibody monotherapy, the combination of *Alistipes shahii* with the anti-PD-1 monoclonal antibody could significantly inhibit the tumor growth.
      *Alistipes shahii* could also significantly reduce the infiltration of Th17 cells and exhausted immune cells (PD-1⁺ cells) in the tumor and increase the CD8⁺ T cells and effector CD8⁺ T cells (GZMB⁺ CD8⁺ T cells) infiltrating the tumor. The combined use of *Alistipes shahii* with the anti-PD-1 monoclonal antibody could further enhance this effect.
      The combined use of the anti-IL-17A monoclonal antibody with the anti-PD-1 monoclonal antibody could also significantly reduce the infiltration of Th17 cells and exhausted immune cells (PD-1⁺ cells) in the tumor and increase the CD8⁺ T cells and effector CD8⁺ T cells (GZMB⁺ CD8⁺ T cells) infiltrating the tumor (as shown in FIG. 4, FIG. 5A, FIG. 5B, FIG. 6, FIG. 7A, and FIG. 7B).
      Further validation revealed that the combination of the anti-IL-17A monoclonal antibody with the anti-PD-1 monoclonal antibody also exhibited a significant tumor-suppressive effect. However, the presence of IL-17A significantly attenuated the sensitizing effect of *Alistipes shahii* on immunotherapy. Therefore, *Alistipes shahii* may promote an anti-tumor immune response by inhibiting Th17 cells and IL-17A-associated signaling pathways (as shown in FIG. 8A, FIG. 8B, FIG. 9A, FIG. 9B, and FIG. 9C).
6. Validation in mouse models of subcutaneous CRC:
   (1) Model construction:
      Six-week-old BALB/c mice were randomly divided into the following four groups:
         a CTRL group, an anti-PD-1 group, a *Butyricimonas virosa* group, and a *Butyricimonas virosa +* anti-PD-1 group.
      Specific treatments for the groups were as follows:
         CTRL group: Mice were intragastrically administered with 200 µL of PBS every other day until an experimental endpoint.
         *Butyricimonas virosa* group: Mice were intragastrically administered with 1 × 10^9 CFU resuspended in 200 µL of PBS every other day until an experimental endpoint.
   (2) Cell injection and tumor model establishment:
      After four rounds of intragastric administration of bacteria or PBS, mice were subcutaneously injected with an MSS CT26 cell line (2,000,000/mouse) to establish a mouse model of subcutaneous CRC.
      When a tumor volume reached 80 mm³, a treatment was started:
      Based on the *Butyricimonas virosa* group, an anti-PD-1 monoclonal antibody (100 µg/mouse, twice weekly until an experimental endpoint) was intraperitoneally injected to create the *Butyricimonas virosa +* anti-PD-1 group.
   (3) Experimental results:
      In BALB/c mice (models of subcutaneous MSS CRC), *Butyricimonas virosa* could enhance the sensitivity of mice to the anti-PD-1 monoclonal antibody therapy. Compared to the anti-PD-1 monoclonal antibody monotherapy, the combination of *Butyricimonas virosa* with the anti-PD-1 monoclonal antibody could significantly inhibit the tumor growth. Notably, the *Butyricimonas virosa* monotherapy also demonstrated significant anti-tumor efficacy (as shown in FIG. 10A, FIG. 10B, and FIG. 10C).
7. Validation in mouse models of orthotopic CRC:
   (1) Model construction:
      Six-week-old BALB/c mice were randomly divided into the following four groups:
      a CTRL group, an anti-PD-1 group, a Butyricimonas virosa group, and a *Butyricimonas virosa +* anti-PD-1 group. Specific treatments for the groups were as follows:
      CTRL group: Mice were intragastrically administered with 200 µL of PBS every other day until an experimental endpoint.
      *Butyricimonas virosa* group: Mice were intragastrically administered with 1 × 10^9 CFU resuspended in 200 µL of PBS every other day until an experimental endpoint.
   (2) Cell injection and tumor model establishment:
      After four rounds of intragastric administration of bacteria or PBS, an MSS CT26-luc cell line (500,000 cells/mouse) was injected into the cecal serosal layer of mice to establish a mouse model of orthotopic CRC.
      A tumor size and an animal body weight were monitored every other day.
      When a tumor volume reached 80 mm³, a treatment was started:
      Based on the *Butyricimonas virosa* group, an anti-PD-1 monoclonal antibody (100 µg/mouse, twice weekly until an experimental endpoint) was intraperitoneally injected to create the *Butyricimonas virosa +* anti-PD-1 group.
   (3) Experimental results:
      In BALB/c mice (mouse models of orthotopic MSS CRC), *Butyricimonas virosa* could enhance the sensitivity of mice to the anti-PD-1 monoclonal antibody therapy. Compared to the anti-PD-1 monoclonal antibody monotherapy, the combination of *Butyricimonas virosa* with the anti-PD-1 monoclonal antibody could significantly inhibit the tumor growth (as shown in FIG. 11A, FIG. 11B, FIG. 11C, FIG. 11D, and FIG. 11E).

The following conclusions can be drawn:
(1) In BALB/c mice (models of subcutaneous MSS CRC), *Alistipes shahii* can enhance the sensitivity of mice to the anti-PD-1 monoclonal antibody therapy. Compared to the anti-PD-1 monoclonal antibody monotherapy, the combination of *Alistipes shahii* with the anti-PD-1 monoclonal antibody can significantly inhibit the tumor growth. In addition, the combination of the anti-IL-17A monoclonal antibody with the anti-PD-1 monoclonal antibody also exhibits a significant tumor-suppressive effect. However, the presence of IL-17A significantly attenuates the sensitizing effect of *Alistipes shahii* on immunotherapy. Therefore, *Alistipes shahii* may promote an anti-tumor immune response by inhibiting IL-17A-associated signaling pathways.
(2) In both C57BL/6 mice (mouse models of MSI-H CRC) and BALB/c mice (mouse models of orthotopic MSS CRC), *Alistipes shahii* can enhance the sensitivity of mice to the anti-PD-1 monoclonal antibody therapy. Compared to the anti-PD-1 monoclonal antibody monotherapy, the combination of *Alistipes shahii* with the anti-PD-1 monoclonal antibody can significantly inhibit the tumor growth.
(3) In BALB/c mice (models of subcutaneous MSS CRC), *Butyricimonas virosa* can enhance the sensitivity of mice to the anti-PD-1 monoclonal antibody therapy. Compared to the anti-PD-1 monoclonal antibody monotherapy, the combination of *Butyricimonas virosa* with the anti-PD-1 monoclonal antibody can significantly inhibit the tumor growth.
(4) In BALB/c mice (mouse models of orthotopic MSS CRC), *Butyricimonas virosa* can enhance the sensitivity of mice to the anti-PD-1 monoclonal antibody therapy. Compared to the anti-PD-1 monoclonal antibody monotherapy, the combination of *Butyricimonas virosa* with the anti-PD-1 monoclonal antibody can significantly inhibit the tumor growth. Notably, the *Butyricimonas virosa* monotherapy also demonstrates significant anti-tumor efficacy.

### Example 3 Identification of gut microbiota-associated metabolites

Screening criteria for the gut microbiota-associated metabolites were as follows:

### Screening model:

### 1. Screening and detection:

Mice bearing subcutaneous MSS CRC tumors were selected. According to different treatments, the experimental mice were divided into the following groups:
PBS intragastric administration group (PBS group);
anti-PD-1 group;
*Alistipes shahii* intragastric administration group *(Alistipes shahii* group);
*Butyricimonas virosa* intragastric administration group *(Butyricimonas virosa* group);
*Alistipes shahii*/*anti-PD-1* monoclonal antibody combined administration group (abbreviated as *Alistipes shahii +* anti-PD-1 group); and
*Butyricimonas* virosa/anti-PD-1 monoclonal antibody combined administration group (abbreviated as *Butyricimonas virosa* + anti-PD-1 group).
3 biological replicates were set for each group. Intestinal contents were collected from the mice bearing subcutaneous MSS CRC tumors, and subjected to full-spectrum metabolomics PLUS analysis by combining untargeted and widely-targeted metabolomics technologies, thus obtaining comprehensive metabolomic profile data for subsequent analysis.

### 2. Supernatant from in vitro bacterial culture:

Four 15 mL centrifuge tubes were prepared, and the following medium and treatments were added, respectively:
10 mL of ATCC 1490 medium (ATCC 1490 group); and
10 mL of ATCC 1490 medium *+ Alistipes shahii* (*Alistipes shahii* culture supernatant group).

The centrifuge tubes were each incubated at 37°C in an anaerobic environment (gas composition: 80% of N₂, 10% of CO₂, and 10% of H₂) for 48 h. Then, a culture supernatant in each group was collected. 2 biological replicates were set for each group. Each sample was subjected to full-spectrum metabolomics PLUS analysis by combining untargeted and widely-targeted metabolomics technologies, thus obtaining comprehensive metabolomic profile data for subsequent analysis. Metabolomics analysis confirmed that *Alistipes shahii* could produce the following metabolites: O-phosphorylethanolamine, 5,6-dihydroxyindole-2-carboxylic acid, and phosphatidylcholine (O-16:0_20:4).

Metabolomics analysis confirmed that *Butyricimonas virosa* could produce the metabolite: 4-acetamidobutyric acid.

Principles of identification and quantification for metabolites by full-spectrum metabolomics PLUS analysis: All sample extracts were equally pooled to produce a QC sample. Untargeted detection was conducted using a liquid chromatography-quadrupole time-of-flight tandem mass spectrometry (LC-QTOF-MS/MS) platform. Accurate identification was conducted based on a self-established standard database (including MS/MS spectra and retention time (RT)) integrated with a Ds-all public database (including databases such as Metlin, HMDB, and KEGG), an artificial intelligence (AI)-predicted library, and MetDNA. Information such as multiple ion pairs and RT of the identified metabolites was extracted, and based on a self-established targeted database, a project-specific new library was constructed. Finally, in all samples, metabolites in the new library were subjected to multiple reaction monitoring (MRM)-based accurate quantification using a Q-Trap instrument platform. The quantification of metabolites was achieved using an MRM mode of triple quadrupole mass spectrometry. In the MRM mode, a first quadrupole first screened precursor ions (parent ions) of a target substance and excluded precursor ions corresponding to other substances, thereby preliminarily eliminating interference. The precursor ions were then fragmented through collision-induced dissociation in a second quadrupole, and accordingly, a series of fragment ions specific to the substance were generated based on structural features of the substance itself. These fragment ions were then further screened by a third quadrupole to select a typical characteristic fragment ion, such that interference from non-target ions was excluded, resulting in accurate quantification and excellent reproducibility. After LC-MS raw data were acquired from different samples, peak areas of the extracted ion chromatographic peaks for all metabolites were each integrated. Subsequently, chromatographic peak integrals of a same metabolite across different samples were corrected to ensure consistency.

### Results:

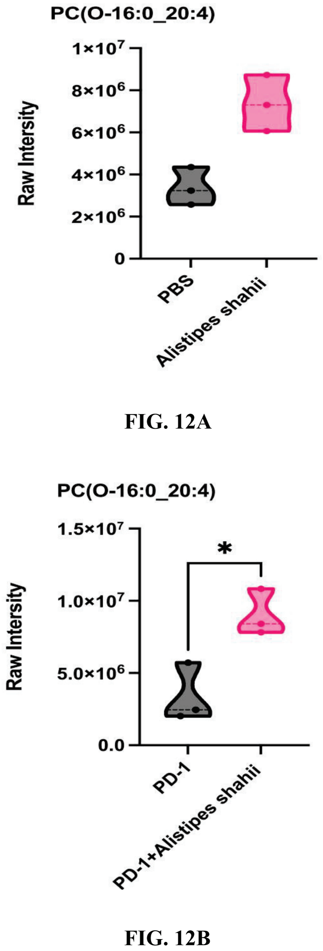

According to the comparison between the *Alistipes shahii* group and the PBS group, and the comparison between the *Alistipes shahii* + anti-PD-1 group and anti-PD-1 group, it was determined that phosphatidylcholine (O-16:0_20:4) was significantly enriched in intestinal contents of mice in both the *Alistipes shahii* group and the *Alistipes shahii* + anti-PD-1 group (Q1: 768.6, Q3: 184.1, and RT: 6.45) (as shown in FIG. 12A, and FIG. 12B).

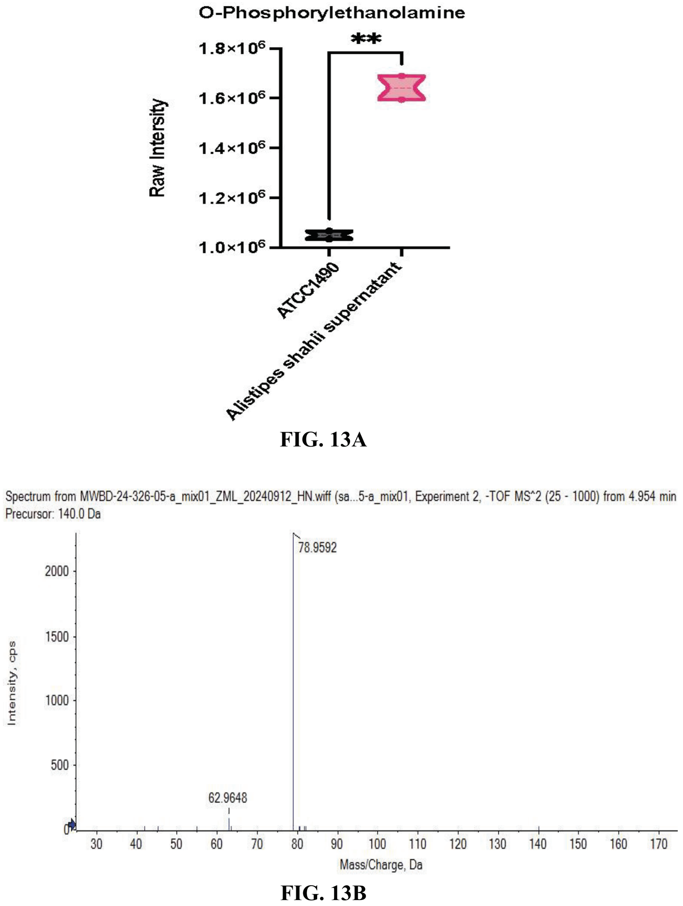

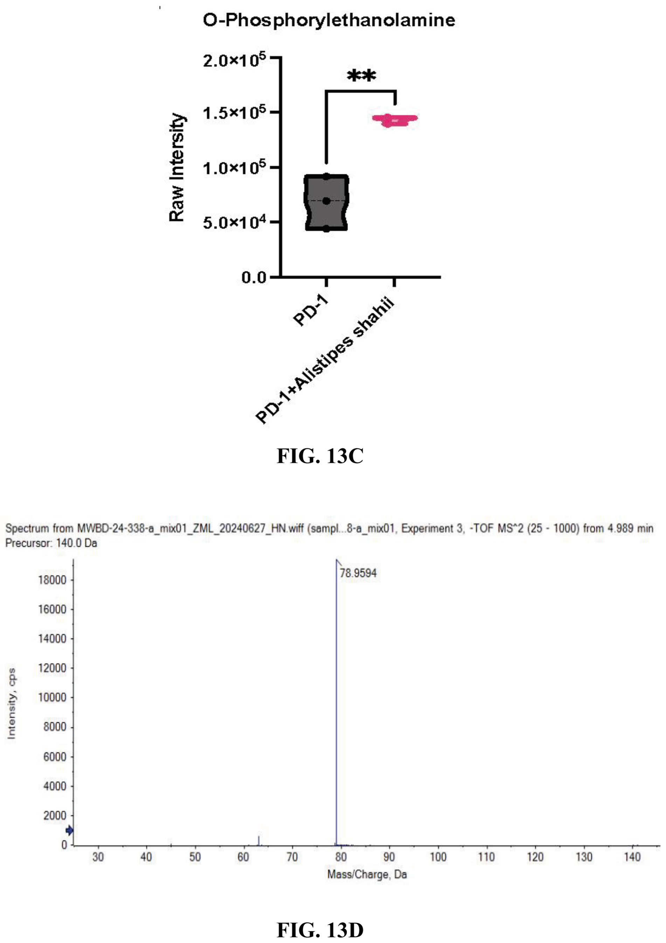

According to the comparison between the *Alistipes shahii* + anti-PD-1 group and the anti-PD-1 group, and the comparison between *the Alistipes shahii* culture supernatant group and the blank ATCC 1490 medium group, it was determined that O-phosphorylethanolamine was significantly enriched in culture supernatants of the *Alistipes shahii* + anti-PD-1 group and the *Alistipes shahii* group (as shown in FIG. 13A, FIG. 13B, FIG. 13C, and FIG. 13D).

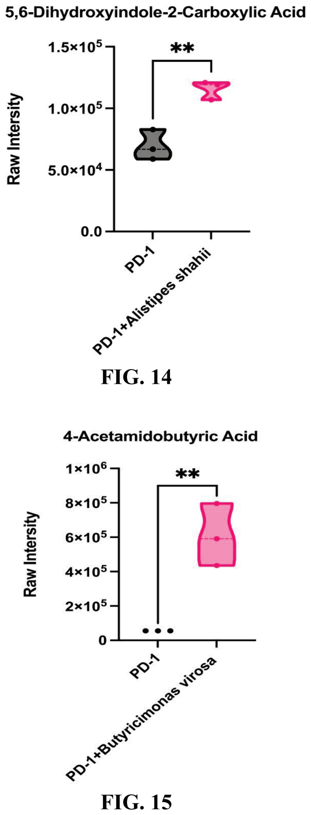

According to the comparison between the *Alistipes shahii* + anti-PD-1 group and the anti-PD-1 group, it was determined that 5,6-dihydroxyindole-2-carboxylic acid was significantly enriched in an intestinal content of the *Alistipes shahii +* anti-PD-1 group (Q1: 192.03, Q3: 148, and RT: 2.72) (as shown in FIG. 14).

According to the comparison between the *Butyricimonas virosa +* anti-PD-1 group and the anti-PD-1 group, it was determined that 4-acetamidobutyric acid was significantly enriched in an intestinal content of the *Butyricimonas virosa +* anti-PD-1 group (Q1: 146.08, Q3: 86, and RT: 1.7) (as shown in FIG. 15).

**Example 4 Identification of an action mechanism of gut microbiota (bulk transcriptomes)**

In this example, bulk transcriptome data of a total of 54 clinical samples from patients enrolled in a clinical trial was submitted for analysis in the present application, including:
15 pre-treatment tumor tissue samples from sensitive (MPR) patients;
16 post-treatment tumor tissue samples from sensitive (MPR) patients;
11 pre-treatment tumor tissue samples from insensitive (non-MPR) patients; and
12 post-treatment tumor tissue samples from insensitive (non-MPR) patients.

### Results:

### 1. KEGG enrichment analysis based on differentially expressed genes:

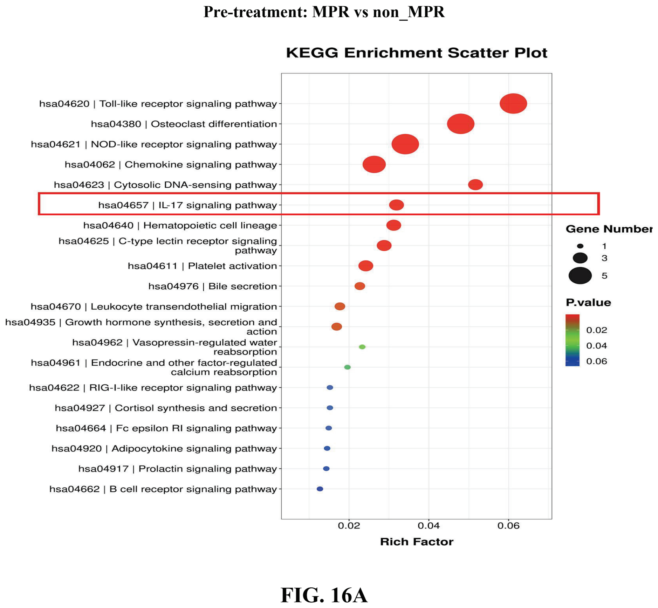

Compared to the non-MPR patients, the IL-17 signaling pathway was significantly down-regulated in pre-treatment tumor tissues of the MPR patients (as shown in FIG. 16A).

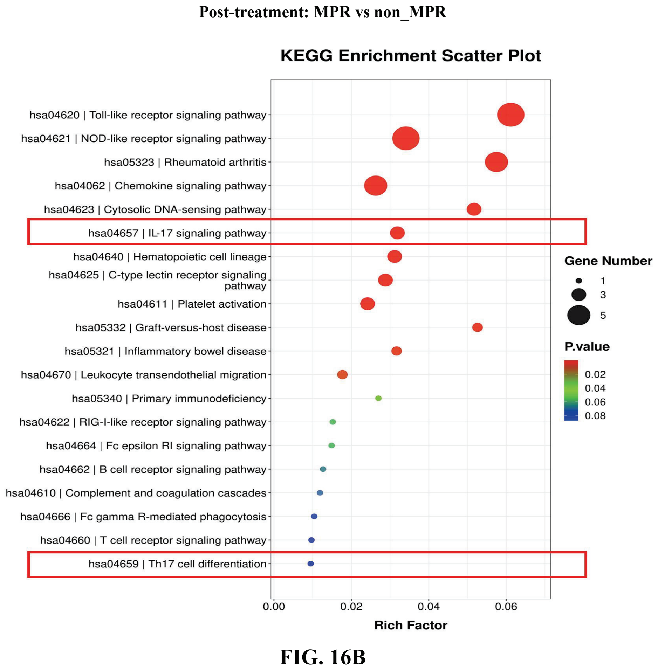

Compared to the non-MPR patients, IL-17 signaling pathway and Th17 cell differentiation pathway were significantly down-regulated in post-treatment tumor tissues of the MPR patients (as shown in FIG. 16B).

### 2. GSEA

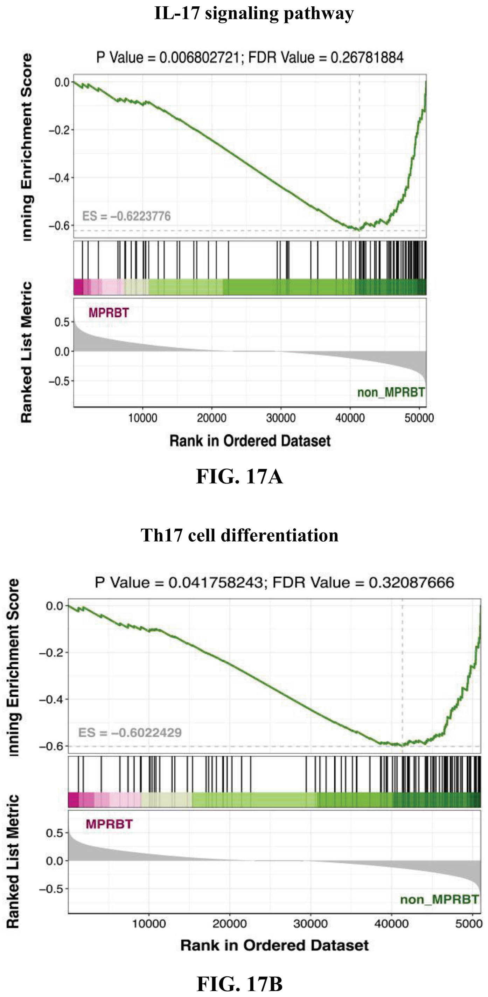

Compared to the non-MPR patients, IL-17 signaling pathway and Th17 cell differentiation pathway were significantly down-regulated in pre-treatment tumor tissues of the MPR patients (as shown in FIG. 17A and FIG. 17B).

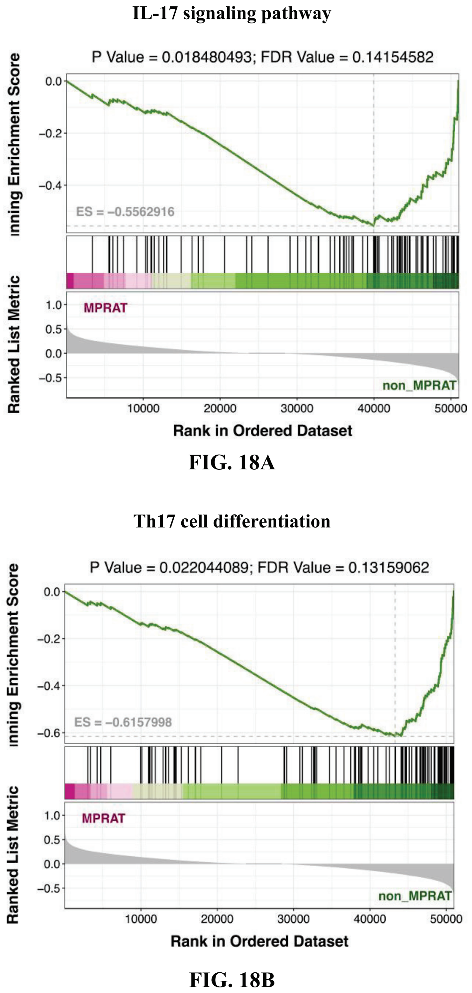

Compared to the non-MPR patients, IL-17 signaling pathway and Th17 cell differentiation pathway were significantly down-regulated in post-treatment tumor tissues of the MPR patients (as shown in FIG. 18A and FIG. 18B).

### Example 5 Influence of gut microbiota metabolites on an IL-17A signaling pathway

### Experimental method:

### 1. Isolation of CD4⁺ T cells from a spleen:

### (1) Preparation of a splenocyte suspension:

A spleen was collected from BALB/c mice, placed in 3 mL to 5 mL of PBS, and ground into a cell suspension.

The cell suspension was transferred to a centrifuge tube, and 0.2 mL of an RPMI 1640 medium was slowly added to cover a surface of the cell suspension.

### (2) Gradient centrifugation:

The gradient centrifugation was conducted at 800 × g for 30 min.

A lymphocyte layer between the medium and a lymphocyte separation solution was collected, washed twice with the RPMI 1640 medium, and then counted for a cell concentration.

### (3) Sorting of CD4⁺ T cells:

The cell concentration was adjusted to 2 × 10⁹/L, and a fluorescein Isothiocyanate (FITC)-labeled anti-CD4 monoclonal antibody was added.

CD4⁺T cells were sorted under sterile conditions by a flow cytometer, and a purity of sorted cells was detected.

### 2. Induction of the differentiation of CD4⁺ T cells into Th17 cells:

### (1) Coating of culture dishes:

An anti-mouse CD3 antibody (3 µg/mL) was added to a 60 × 15 mm plastic culture dish and incubated at 37°C for 2 h or at 4°C overnight.

After the incubation, washing was conducted three times with sterile PBS.

### (2) Standard culture conditions:

CD4⁺ T cells were inoculated at a density of 2 × 10⁶/mL into pre-coated culture dishes, and the following induction factors were added, respectively:
anti-mouse CD28 (5 µg/mL);
Recombinant mouse IL-6 (100 ng/mL);
Recombinant mouse TGF-β1 (1 ng/mL);
Recombinant mouse IL-1β (10 ng/mL);
Recombinant mouse IL-23 (5 ng/mL);
anti-mouse IL-4 (10 µg/mL);
anti-mouse IFN-γ (10 µg/mL); and
O-phosphorylethanolamine and 5,6-dihydroxyindole-2-carboxylic acid, which were the gut microbiota metabolites of the present application, were respectively used in two treatment groups.

The cells were cultured at 37°C and 5% CO₂ for 4 d.

### 3. Experimental results:

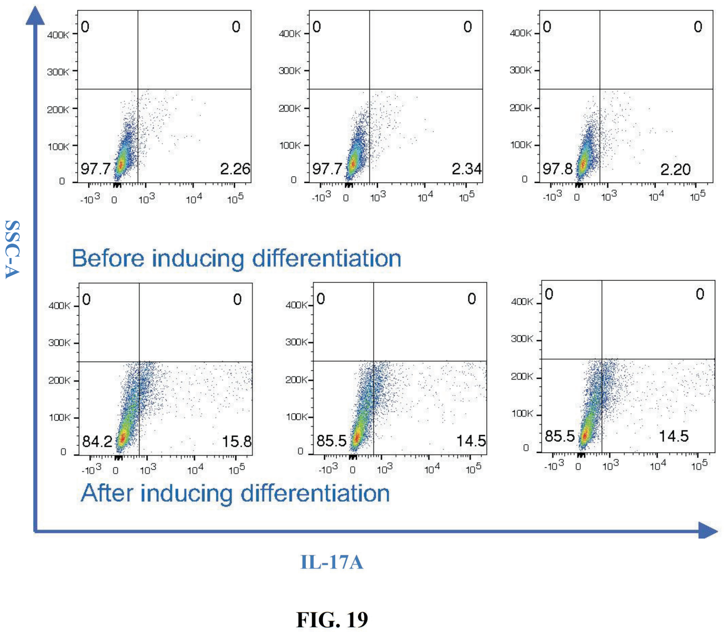

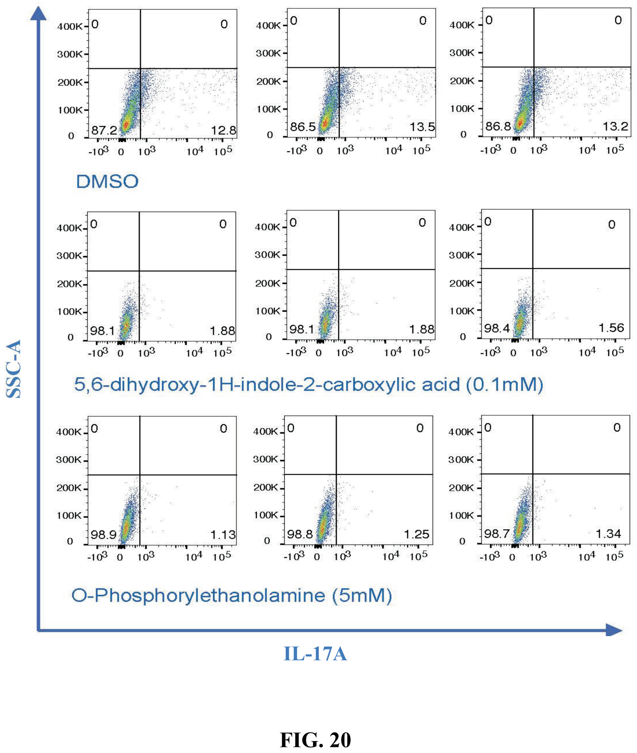

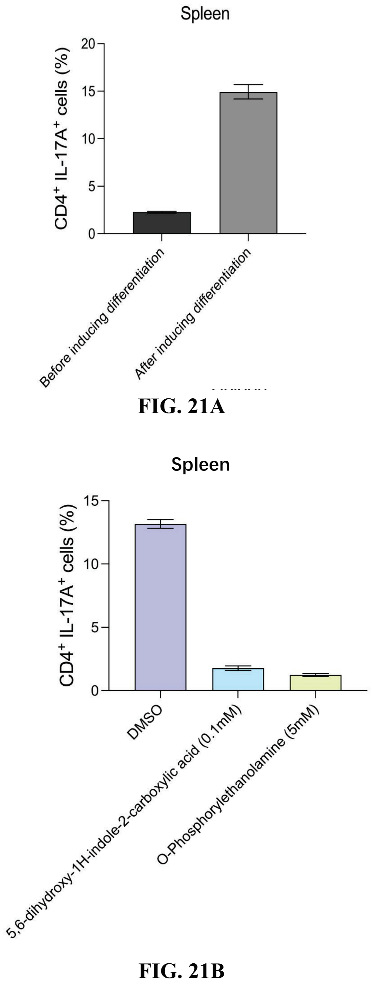

As shown in FIG. 19, FIG. 20, FIG. 21A and FIG. 21B, compared with undifferentiated cells, a proportion of CD4⁺ IL-17A⁺ T cells significantly increased after the induced differentiation. Compared with the dimethyl sulfoxide (DMSO) control group, the treatment with 5,6-dihydroxyindole-2-carboxylic acid (0.1 mM) and the treatment with O-phosphorylethanolamine (5 mM) both significantly reduced the proportion of CD4⁺ IL-17A⁺ T cells. O-phosphorylethanolamine and 5,6-dihydroxyindole-2-carboxylic acid each suppressed the differentiation of naive CD4⁺ T cells into Th17 cells to reduce IL-17A secreted by Th17 cells, thereby down-regulating the activity of the IL-17A signaling pathway.

Based on these findings, *Alistipes shahii* effectively suppresses the IL-17A signaling pathway through its metabolites O-phosphorylethanolamine and 5,6-dihydroxyindole-2-carboxylic acid, thereby ameliorating the tumor microenvironment and enhancing the anti-tumor efficacy of immunotherapy. This mechanism further elucidates the significant role of the gut microbiota metabolites in modulating the immune microenvironment and improving the sensitivity to tumor immunotherapy.

### Example 6 Validation experiment on gut microbiota metabolites

### 1. Construction of subcutaneous CRC mouse models:

Six-week-old BALB/c mice were randomly divided into the following six groups: a CTRL (PBS) group, a 5,6-dihydroxyindole-2-carboxylic acid (DICA) group, an O-phosphorylethanolamine (OP) group, an anti-PD-1 group, a 5,6-dihydroxyindole-2-carboxylic acid (DICA) + anti-PD-1 group, and an O-phosphorylethanolamine (OP) + anti-PD-1 group.

Specific treatments for the groups were as follows:
CTRL (PBS) group: Mice were intragastrically administered with 200 µL of PBS every other day until an experimental endpoint.

5,6-dihydroxyindole-2-carboxylic acid (DICA) group: Based on a mouse body weight, each mouse was intragastrically administered with 20 mg/kg of 5,6-dihydroxyindole-2-carboxylic acid every other day until an experimental endpoint.

O-phosphorylethanolamine (OP) group: Based on a mouse body weight, each mouse was intragastrically administered with 20 mg/kg of O-phosphorylethanolamine every other day until an experimental endpoint.

Anti-PD-1 group: When a tumor volume reached 80 mm³, an anti-PD-1 monoclonal antibody was intraperitoneally injected (100 µg/mouse, twice weekly until an experimental endpoint).

5,6-dihydroxyindole-2-carboxylic acid (DICA) + anti-PD-1 group: Based on the 5,6-dihydroxyindole-2-carboxylic acid (DICA) group, when a tumor volume reached 80 mm³, an anti-PD-1 monoclonal antibody was intraperitoneally injected (100 µg/mouse, twice weekly until an experimental endpoint).

O-phosphorylethanolamine (OP) + anti-PD-1 group: Based on the O-phosphorylethanolamine (OP) group, when a tumor volume reached 80 mm³, an anti-PD-1 monoclonal antibody was intraperitoneally injected (100 µg/mouse, twice weekly until an experimental endpoint).

### 2. Cell injection and tumor model establishment:

After four rounds of intragastric administration of metabolites or PBS, mice were subcutaneously injected with an MSS CT26 cell line (2,000,000/mouse) to establish a mouse model of subcutaneous CRC.

### 3. Experimental results:

In BALB/c mice (models of subcutaneous MSS CRC), both O-phosphorylethanolamine and 5,6-dihydroxyindole-2-carboxylic acid could enhance the sensitivity of the mice to the anti-PD-1 monoclonal antibody therapy.

Compared with the anti-PD-1 monoclonal antibody monotherapy, the combination of either O-phosphorylethanolamine or 5,6-dihydroxyindole-2-carboxylic acid with the anti-PD-1 monoclonal antibody significantly inhibited the tumor growth.

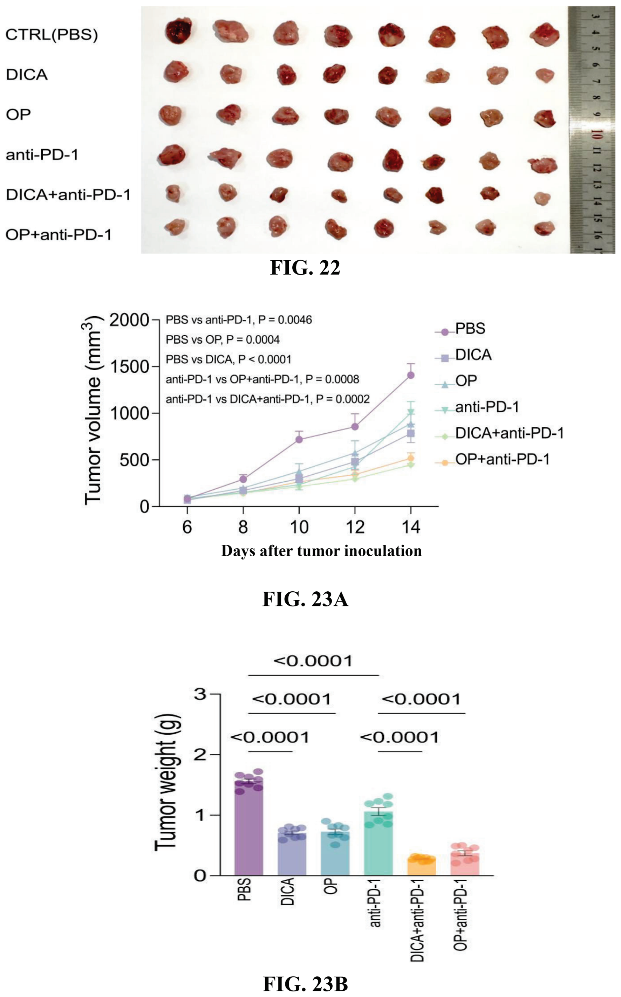

Notably, the administration of O-phosphorylethanolamine or 5,6-dihydroxyindole-2-carboxylic acid alone also demonstrated a significant anti-tumor effect (as shown in FIG. 22, FIG. 23A, and FIG. 23B).

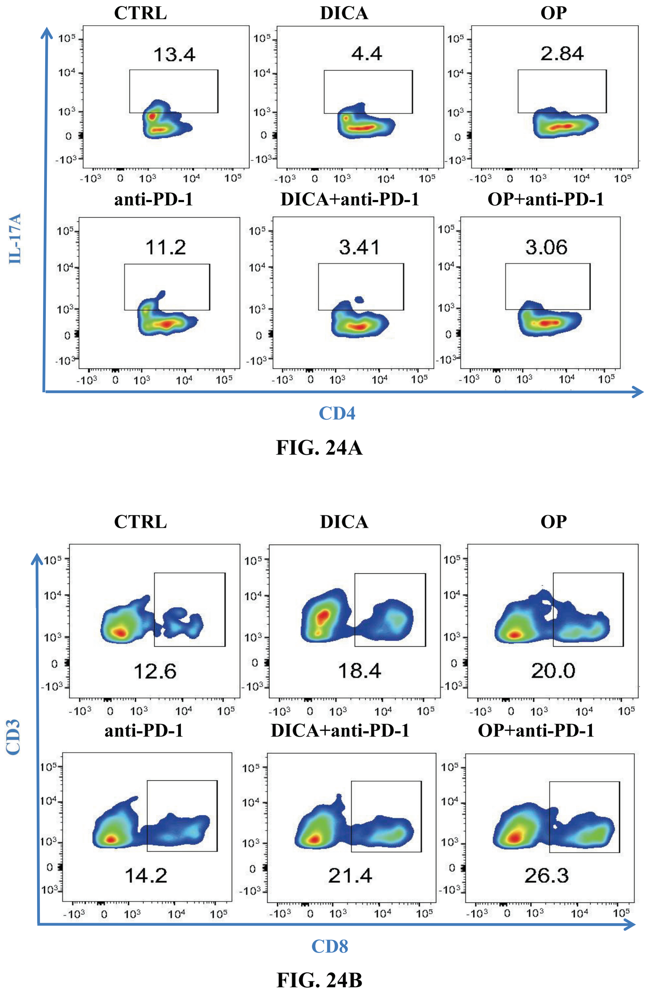

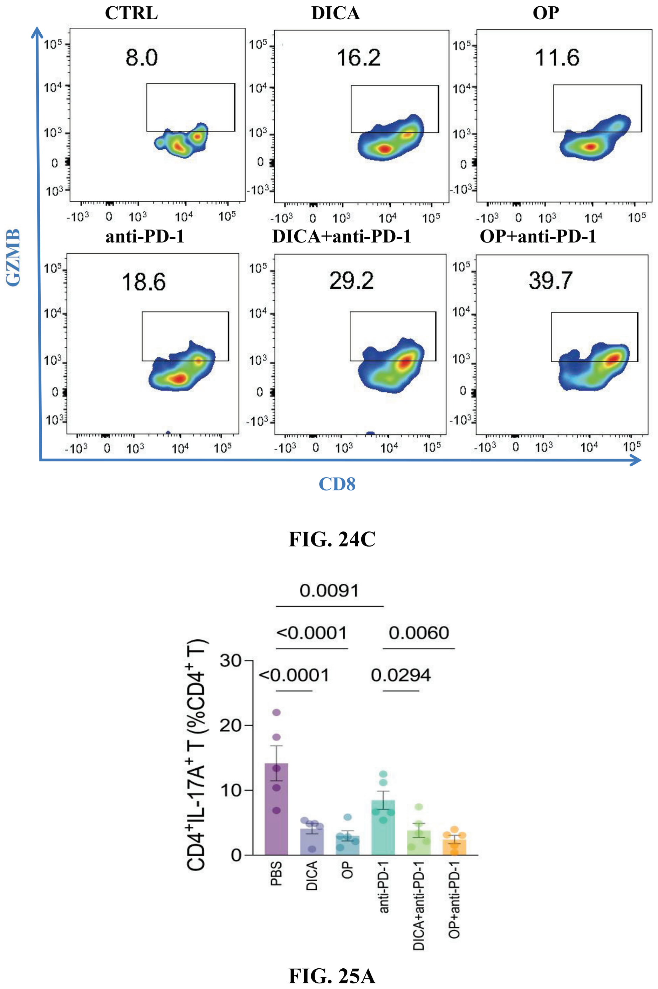

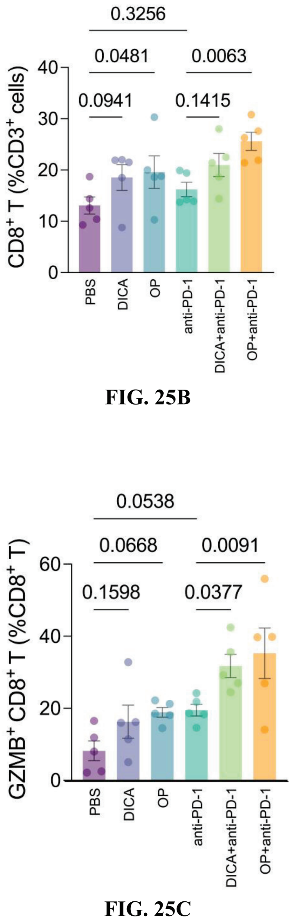

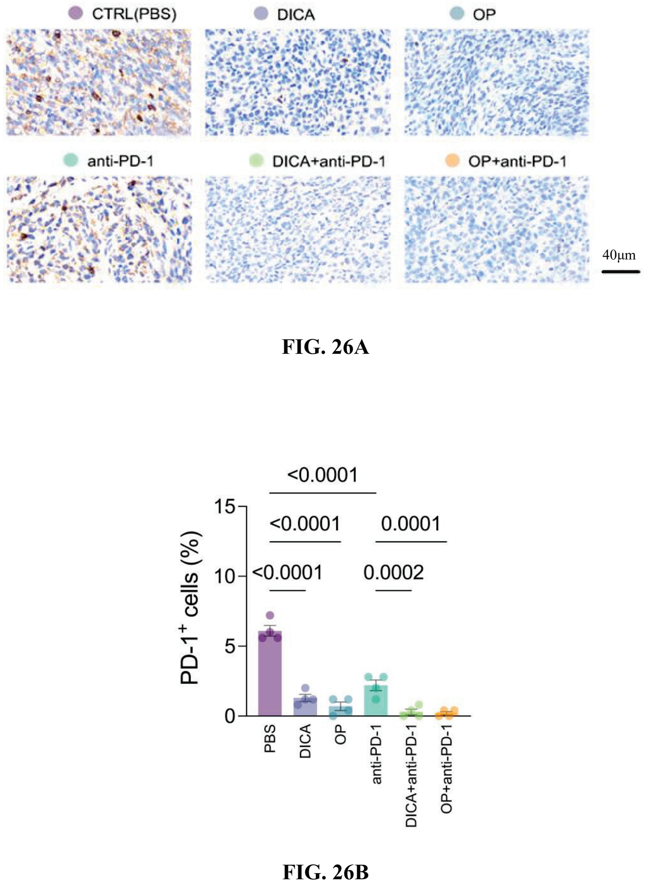

Both O-phosphorylethanolamine and 5,6-dihydroxyindole-2-carboxylic acid could significantly reduce the infiltration of Th17 cells and exhausted immune cells (PD-1⁺ cells) in the tumor and enhance the infiltration and functionality of CD8⁺ T cells in the tumor (as shown in FIG. 24A, FIG. 24B, FIG. 24C, FIG. 25A, FIG. 25B, FIG. 25C, FIG. 26A, and FIG. 26B).

According to the aforementioned experiments, the gut microbiota or the metabolite thereof in the present application can suppress the secretion of IL-17 by inhibiting the differentiation of naive CD4⁺ T cells into Th17 cells, and ameliorate the immunosuppressive status in the tumor microenvironment, thereby enhancing the sensitivity of MSS CRC patients to immunotherapy.

Moreover, the gut microbiota of the present application can significantly reduce the infiltration of Th17 cells and exhausted immune cells (PD-1⁺ cells) in the tumor and increase the CD8⁺ T cells and effector CD8⁺ T cells (GZMB⁺ CD8⁺ T cells) infiltrating the tumor. The combined use of the gut microbiota (such as *Alistipes shahii)* with the anti-PD-1 monoclonal antibody can further enhance this effect.

The combined use of the anti-IL-17A monoclonal antibody with the anti-PD-1 monoclonal antibody can also significantly reduce the infiltration of Th17 cells and exhausted immune cells (PD-1⁺ cells) in the tumor and increase the CD8⁺ T cells and effector CD8⁺ T cells (GZMB⁺ CD8⁺ T cells) infiltrating the tumor. Therefore, the gut microbiota (such as *Alistipes shahii)* may suppress the infiltration of Th17 cells in the tumor to promote the infiltration and functionality of CD8⁺ T cells in the tumor, thereby facilitating an anti-tumor immune response.

The aforementioned metabolites all exhibit a significant anti-tumor effect when used alone. These metabolites all can significantly reduce the infiltration of Th17 cells and exhausted immune cells (PD-1⁺ cells) in the tumor, and enhance the infiltration and functionality of CD8⁺ T cells in the tumor.

Finally, it should be noted that the above examples are provided merely to describe the technical solutions of the present application, rather than to limit the protection scope of the present application. Although the present application is described in detail with reference to preferred examples, a person of ordinary skill in the art should understand that modifications or equivalent replacements may be made to the technical solutions of the present application without departing from the spirit and scope of the technical solutions of the present application.

## Claims

1. At least one agent selected from the group consisting of:
(a) a gut microbiota or a metabolite thereof, and
(b) an antibody or a derivative thereof,
for use in immunotherapy of colorectal cancer (CRC),
wherein the gut microbiota comprises at least one selected from the group consisting of *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii;* and
wherein the antibody comprises a monoclonal antibody targeting an interleukin-17A (IL-17A) signaling pathway, a monoclonal antibody targeting CRC, or or a combination thereof.

2. The at least one agent for use according to claim 1, wherein the gut microbiota comprises at least two selected from the group consisting of *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii.*

3. A composition for use in immunotherapy of CRC, wherein the composition comprises:
(1) at least one agent selected from the group consisting of:
(a) a gut microbiota or a metabolite thereof, and
(b) an antibody or a derivative thereof; and
(2) a product for treating CRC,
wherein the gut microbiota comprises at least one selected from the group consisting of *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii;* and
wherein the antibody comprises a monoclonal antibody targeting an interleukin-17A (IL-17A) signaling pathway, a monoclonal antibody targeting CRC, or or a combination thereof.

4. The composition for use according to claim 3, wherein the product for treating CRC comprises at least one selected from the group consisting of a microbial strain, an immune checkpoint inhibitor (ICI), an immune activator, a cancer vaccine, an immune cell therapy drug, and a cytokine therapy drug;
preferably, the microbial strain comprises at least one selected from the group consisting of *Lactobacillus rhamnosus, Lactobacillus reuteri, Lactobacillus johnsonii,* and *Bifidobacterium.*

5. The composition for use according to claim 3, wherein the at least one agent comprises the monoclonal antibody targeting the IL-17A signaling pathway, and the product for treating CRC comprises an immune checkpoint inhibitor.

6. The at least one agent according to claim 1 for use in improving or predicting sensitivity to an immune checkpoint inhibitor.

7. The at least one agent for use according to any one of claims 1-2 and 6, or the composition for use according to any one of claims 3-5, wherein the metabolite of the gut microbiota comprises at least one selected from the group consisting of O-phosphorylethanolamine, 5,6-dihydroxyindole-2-carboxylic acid, phosphatidylcholine (O-16:0_20:4), and 4-acetamidobutyric acid;
preferably, a metabolite of *Alistipes shahii* comprises at least one selected from the group consisting of O-phosphorylethanolamine, 5,6-dihydroxyindole-2-carboxylic acid, and phosphatidylcholine (O-16:0_20:4); and/or a metabolite of *Butyricimonas virosa* comprises 4-acetamidobutyric acid.

8. The at least one agent for use according to claim 6, or the composition for use according to claim 4 or claim 5, wherein the immune checkpoint inhibitor comprises an anti-programmed cell death protein 1 (PD-1) antibody, an anti-programmed cell death-ligand 1 (PD-L1) antibody, or a combination thereof.

9. The at least one agent for use according to any one of claims 1-2 and 6-8, or the composition for use according to any one of claims 3-5 and 7-8, wherein the CRC comprises microsatellite stability (MSS) CRC and microsatellite instability (MSI) CRC;
preferably, dosage forms of (1) the at least one agent and optionally (2) the product for treating CRC are each independently selected from the group consisting of liquid, gas, solid, and semi-solid dosage forms;
preferably, the dosage form further comprises a pharmaceutically acceptable excipient.

10. A pharmaceutical composition, comprising:
(1) at least one agent selected from the group consisting of:
(a) a gut microbiota or a metabolite thereof, and
(b) an antibody or a derivative thereof; and
(2) a product for treating CRC,
wherein the gut microbiota comprises at least one selected from the group consisting of *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii;* and
wherein the antibody comprises a monoclonal antibody targeting an IL-17A signaling pathway, a monoclonal antibody targeting CRC, or a combination thereof.

11. The pharmaceutical composition according to claim 10, wherein the product for treating CRC comprises at least one selected from the group consisting of a microbial strain, an immune checkpoint inhibitor, an immune activator, a cancer vaccine, an immune cell therapy drug, and a cytokine therapy drug.

12. The pharmaceutical composition according to claim 11, wherein the product for treating CRC comprises the immune checkpoint inhibitor; and preferably, the immune checkpoint inhibitor comprises an anti-PD-1 antibody, an anti-PD-L1 antibody, or a combination thereof.

13. The pharmaceutical composition according to claim 11, wherein the product for treating CRC comprises the microbial strain; and preferably, the microbial strain comprises at least one selected from the group consisting of *Lactobacillus rhamnosus, Lactobacillus reuteri, Lactobacillus johnsonii,* and *Bifidobacterium.*

14. The pharmaceutical composition according to any one of claims 11-13 for use in increasing sensitivity of CRC to an immunotherapy drug, wherein the product for treating CRC comprises an immune checkpoint inhibitor;
preferably, the increasing sensitivity of CRC to an immunotherapy drug comprises increasing sensitivity to the immune checkpoint inhibitor;
preferably, the increasing sensitivity of CRC to an immunotherapy drug further comprises increasing sensitivity to an immune cell therapy drug and/or a cytokine therapy drug.

15. At least one agent selected from the group consisting of:
(a) a gut microbiota or a metabolite thereof, and
(b) an antibody or a derivative thereof,
for use in inhibiting an IL-17A signaling pathway,
wherein the gut microbiota comprises at least one selected from the group consisting of *Alistipes shahii, Butyricimonas virosa, Desulfovibrio piger, Alistipes putredinis, Odoribacter splanchnicus, Faecalibacterium prausnitzii, Phocaeicola plebeius, Eubacterium rectale, Oscillospiraceae bacterium,* and *Alistipes onderdonkii;*
wherein the metabolite of the gut microbiota comprises at least one selected from the group consisting of O-phosphorylethanolamine, 5,6-dihydroxyindole-2-carboxylic acid, phosphatidylcholine (O-16:0_20:4), and 4-acetamidobutyric acid; and
wherein the antibody comprises a monoclonal antibody targeting an IL-17A signaling pathway, a monoclonal antibody targeting CRC, or a combination thereof.
